(19) Europäisches Patentamt
European Patent Office
Office européen des brevets

(11) Veröffentlichungsnummer: **0 575 976 A1**

(12) # EUROPÄISCHE PATENTANMELDUNG

(21) Anmeldenummer: **93110009.3**

(22) Anmeldetag: **23.06.93**

(51) Int. Cl.$^{5}$: **A61K 47/48**, C08B 37/00

(30) Priorität: **25.06.92 DE 4220736**

(43) Veröffentlichungstag der Anmeldung: **29.12.93 Patentblatt 93/52**

(84) Benannte Vertragsstaaten: **AT BE CH DE DK ES FR GB GR IE IT LI LU MC NL PT SE**

(71) Anmelder: **MEDICE Chem.-Pharm. Fabrik Pütter GmbH & Co. KG
Kuhloweg 37-39
D-58638 Iserlohn(DE)**

(72) Erfinder: **Szejtli, Jozsef, Prof.
c/o Fa. Cyclolab Laboratories,
Postfach 17
H-1525 Budapest(HU)**
Erfinder: **Pütter, Sigurd, Dr.
c/o MEDICE,
Kuhloweg 37-39
D-5860 Iserlohn(DE)**

(74) Vertreter: **Reinhard, Skuhra, Weise
Postfach 44 01 51
D-80750 München (DE)**

(54) **Einschlusskomplexe aus polymerisierten Cyclodextrinen mit pharmazeutisch aktiven Wirkstoffen.**

(57) Die Erfindung offenbart Einschlußkomplexe aus polymerisierten Cyclodextrinen mit pharmazeutisch aktiven Wirkstoffen, die Herstellung dieser Einschlußkomplexe sowie die Verwendung der Einschlußkomplexe.

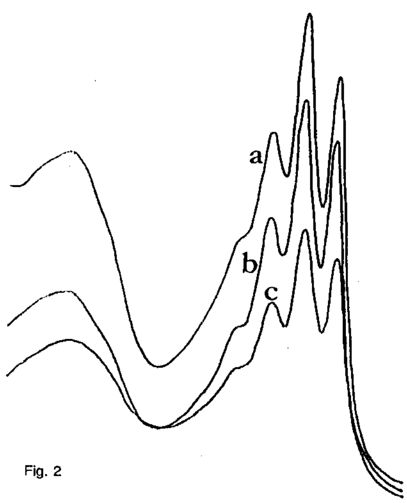


Fig. 2

UV-Spektren von Nystatin, aufgelöst in $\beta$-Cyclodextrinpolymer
a: unsubstituiertes Polymer
b: methyliertes Polymer
c: $\gamma$-Cyclodextrin komplexiert als Referenz

EP 0 575 976 A1

Die Erfindung betrifft Einschlußkomplexe aus polymerisierten Cyclodextrinen mit pharmazeutisch aktiven Wirkstoffen, die Herstellung dieser Einschlußkomplexe sowie die Verwendung der Einschlußkomplexe.

Aus der US-PS 4 225 580 sind ein Verfahren zur Reinigung eiternder Hautoberflächen, Wunden und Schleimhäute und Mittel zur Durchführung dieses Verfahrens bekannt. Diese Druckschrift betrifft wasserunlösliche hydrophile Polymere, beispielsweise Dextrane, die mit Epichlorhydrin vernetzt sind , polymere oder polymerisierte Kohlenhydrate oder vernetzte Zuckeralkohole sowie deren Derivate, die Carboxyl-, Sulfonsäure- und/oder Aminogruppen enthalten, und ihre Verwendung zur Reinigung offener infizierter Stellen. Diese Materialien verhalten sich wie Schwämme, die das Exudat der Wunde aufsaugen. Diese Drainage bewirkt eine beschleunigte Heilung und verhindert eine Überinfektion. Der Heilungsprozeß kann weiter durch den Einbau von Iod in die quellende Matrix verbessert werden (Neue topische Wundtherapie Iodosorb, Symp. am 22. Januar 1983 in München, hrsg. von M. Fisher, J.A. Fax, New York : Schattauer, 1983).

In der ungarischen Patentanmeldung 2599/87 wird ein Wundpuder mit einer kontrollierten Iod-Freisetzung beschrieben, der ein Cyclodextrinpolymer enthält. Dieser Iod-freisetzende Wundpuder wurde hergestellt durch Vermischen eines Cyclodextrinpolymeren und einer Lösung aus Iod und Alkaliiodid und/oder -Bromid und anschließendem Entfernen des Lösungsmittels. Die Drainagewirkung des quellenden Polymers wird durch die desinfizierende Wirkung des Iods ergänzt. Die Iodfreisetzung wird durch das Verhältnis von Iodid zu Bromid kontrolliert, wobei das Iodid die Freisetzung verlangsamt, während das Bromid diese beschleunigt.

Im US-Patent 4 774 329 (1987) wird ein Cyclodextrinpolymer als Mittel zur kontrollierten Freisetzung für Cetylpyridiniumchlorid beschrieben. Diese Patentschrift beschreibt einen Komplex aus Cyclodextrin oder einem Cyclodextrinpolymeren oder einem modifizierten Cyclodextrin und Cetylpyridiniumchlorid zur Behandlung von Wunden oder anderen Oberflächen. Diese Komplexe werden in Form einer Lotion oder Salbe oder auf einem Verband verwendet. Die Verwendung des Cyclodextrinpolymeren direkt auf der Wundoberfläche wird hier nicht beschrieben; weiterhin wird vom Drainageeffekt des Polymeren nicht Gebrauch gemacht.

In diesen zwei zuletzt beschriebenen Druckschriften ist das Iod und das Cetylpyridiniumchlorid in die Cyclodextrinhohlräume im Innern der Polymermatrix eingeschlossen und die Freisetzungsgeschwindigkeit wird durch die Stabilität dieser Komplexe bestimmt.

Es ist eine Aufgabe der Erfindung, pharmazeutische Zubereitungen bereitzustellen, die eine kontrollierte Freisetzung von Wirkstoffen zeigen.

Diese Aufgabe wird erfindungsgemäß dadurch gelöst, daß Einschlußkomplexe aus polymerisierten, unlöslichen und in Wasser quellbaren Cyclodextrinen und/oder deren Derivaten mit Desinfizientia, Antiseptika, Antimykotika, Antiphlogistika, Antibiotika oder Anästhetika oder deren Mischungen als Wirkstoffe bereitgestellt werden.

Die erfindungsgemäßen Komplexe ermöglichen eine kontrollierte Freisetzung der Wirkstoffe.

Die Erfindung betrifft somit pharmazeutische Zubereitungen mit einer kontrollierten Freisetzung von Wirkstoffen, die eine Kombination eines Wirkstoffs und eines quellbaren Cyclodextrinpolymeren enthalten, wobei der Wirkstoff ein desinfizierendes oder antiseptisches oder entzündungshemmendes Mittel oder ein Antibiotikum oder ein Anästhetikum oder ein Antimykotikum ist und das Cyclodextrinpolymer ein $\alpha$-, $\beta$- oder $\gamma$-Cyclodextrinpolymer ist, welches mit einem bifunktionellen Reagenz zu einem dreidimensionalen Netz vernetzt und mit ionischen und/ oder nicht-ionischen Gruppen chemisch modifiziert sein kann. Die Drainagewirkung des quellbaren Cyclodextrinpolymeren wird durch die Wirkstoffwirkung ergänzt, wodurch ein beschleunigter Heilungsprozeß erfolgt.

Erfindungsgemäß wurde weiterhin gefunden, daß die Freisetzungsgeschwindigkeit durch die Substitution der Cyclodextrine mit verschiedenen ionischen und nicht-ionischen Gruppen erhöht oder verringert werden kann, indem der Substitutionsgrad und das Wirkstoff-/Polymer-Verhältnis verändert wird.

Erfindungsgemäß kann die Wirkstofffreisetzung in folgender Weise reguliert werden: Durch

a) die Veränderung des Wirkstoff/CD-Verhältnisses und/oder
b) durch die Veränderung des Substituenten und/oder
c) durch den Substitutionsgrad.

a) Veränderung des Wirkstoff/CD-Verhältnisses

Bei der Auswahl des Wirkstoff/CD-Molverhältnisses ist die Komplexstabilitätskonstante zu berücksichten. Wenn der Wirkstoff-Cyclodextrin-Komplex eine große Stabilitätskonstante aufweist, z.B. bei Jod, Progesteron, Dexamethasonacetat usw. ($K_{ass}>10^4$), werden nur wenige Prozent des freien Wirkstoffs aus dem Polymerkomplex freigesetzt, wodurch sich eine langsame Auflösungsgeschwindigkeit ergibt. In diesem

Fall kann die gewünschte Wirkstofffreisetzungsgeschwindigkeit erhalten werden durch ein Verhältnis Wirkstoff:CD-Ringeinheiten von 2-5:1. Der große Überschuß an Wirkstoffmolekülen, die nur in den von den Quervernetzungen gebildeten Hohlräumen absorbiert oder an die Oberfläche adsorbiert sind, wird relativ schnell freigesetzt. Die Summe dieser zwei Prozesse bildet die Wirkstofffreisetzungsgeschwindigkeit. Wenn der Wirkstoff-Cyclodextrin-Komplex durch eine Stabilitätskonstante von $10^3$-$10^4$ gekennzeichnet ist, ist ein Wirkstoff:CD-Verhältnis von 1-2:1 vorteilhaft. In diesem Fall ist die Mehrzahl der Wirkstoffmoleküle im Innern der CD-Ringe lokalisiert. Bei Kontakt mit Wasser oder einer wäßrigen Lösung wir aufgrund des durch die Stabilitätskonstante gekennzeichneten Komplexgleichgewichtes ein größerer Teil des Wirkstoffs aus dem Komplex freigesetzt.

Wenn der Wirkstoff-Cyclodextrin-Komplex eine geringe Stabilitätskonstante ($K_{ass}<10^2$) aufweist, ist das Wirkstoff:CD-Molverhältnis bevorzugterweise geringer als 1:1, bevorzugt 0,01-0,5:1. Nur dieses geringe Wirkstoff/CD-Verhältnis kann eine Verzögerung in der Wirkstofffreisetzung bewirken. Bei Einstellung dieses geringen Verhältnisses wird nur ein Teil des Wirkstoffs bei Kontakt mit Wasser oder einer wäßrigen Lösung freigesetzt, da das Komplexgleichgewicht durch den Überschuß an Cyclodextrinen verschoben ist.

Um im letzteren Fall die erforderliche Freisetzungsgeschwindigkeit zu erhalten, würde der Gehalt an aktivem Wirkstoff im Polymer so stark abnehmen, daß er nicht mehr das erforderliche Maß erreicht. In diesem Fall wird durch die Wahl eines geeigneten Substituenten zusammen mit dem geeigneten Substitutionsgrad die erfindungsgemäße Aufgabe gelöst.

b) Veränderung des Substituenten

Wenn die Wirt-Gast-Interaktion nicht stark genug ist, können ionische Wechselwirkungen und hydrophobe Wechselwirkungen den Effekt der Komplexbildung ergänzen. Im Falle anionischer Wirkstoffe, z.B. von Ethacridinlactat, Methylenblau, Lidocain, Cetylpyridiniumsalzen, Lidocain, Benzocain, Chlortetracyclin, Chlorprocain und Miconazol, kann ein mit katonischen Gruppen substituiertes Polymer die Auflösung des Wirkstoffs verzögern. Carboxylgruppen, z.B. Carboxymethyl, Carboxyethyl, Carboxypropyl, Carboxybutyl, bevorzugt Carboxymethylgruppen, und Sulphoalkylgruppen, z.B. Sulphoethyl, Sulphopropyl, Sulphobutyl, bevorzugt Sulphopropylgruppen, können dem Polymer einen sauren Charakter verleihen, wodurch die basischen Wirkstoffe ausreichend zurückgehalten werden.

Im Fall kationischer Wirkstoffe, z.B. Ibuprofen, Flurbiprofen, Indomethacin, Salicylsäure und Fumagillin, können die mit kationischen Gruppen substituierten Polymere die Auflösung des Wirkstoffs verzögern. Aminogruppen, bevorzugt Aminoalkylgruppen, z.B. Aminomethyl, Aminoethyl, Aminopropyl, Aminobutylgruppen, aber auch Dialkylaminogruppen, z.B. Dimethylamino, Diethylamino, bevorzugt Dimethylaminoethyl und Diethylaminoethylgruppen, können dem Polymer einen basischen Charakter verleihen, wodurch eine ausreichende Sorption der sauren Wirkstoffe sichergestellt ist.

Bei amphoteren Wirkstoffen mit sowohl basischen als auch sauren Gruppen, z.B. Nystatin, Amphotericin, Eosin, Triphenylmethanfarbstoffen, Oxacillin usw., sind Polymere mit sauren oder basischen Gruppen die geeigneten Sorbentien.

Bei Wirkstoffen mit großen hydrophoben Gruppen, z.B. Steroiden wie Hydrocortison, Beclomethason, Triamcinolon und Tolnaphtat, Amphotericin, Fungisterol usw., bewirken mit liphophilen Gruppen substituierte Polymere eine verzögerte Wirkstofffreisetzung. Erfindungsgemäß sind hier insbesondere die folgenden Gruppen bevorzugt: Alkyl (Methyl, Ethyl, Propyl, Isopropyl, Butyl, Isobutyl, Tert. Butyl, Pentyl, Isopentyl, Hexyl, Heptyl, Octyl, Nonyl, Decyl usw.), Hydroxyalkyl (Hydroxyethyl, Hydroxypropyl, Hydroxyhexyl usw.), Aryl (Phenyl, Naphtyl, Biphenyl, Anthracyl usw.), Alkyaryl (Tolyl, Xylyl, Ethylphenyl, Propylphenyl, Tert. Butylphenyl usw.), Aralkyl (Benzyl, Phenethyl, Benzhydryl, Trityl usw.), Heteroaryl (Piridyl, Picolyl, Bipyridyl, Collidyl usw.), Acyl (Formyl, Acetyl, Propionyl, Butiryl, Isobutiryl, Valeryl, Isovaleryl, Stearyl, Oleyl, Palmitoyl usw.), (Methylsulphonyl, Ethylsulphonyl, Hexylsulphonyl, Dodecylsulphonyl, Methylthio, Butylthio, Hexylthio usw.), Silyl (Trimethylsilyl, Tert. Butyldimethylsilyl usw.)

c) Substitutionsgrad

Die Veränderung des Substitutionsgrades ist ebenfalls ein Mittel, um die Auflösungsgeschwindigkeit zu kontrollieren. Je höher der Substitutionsgrad, desto geringer die Freisetzungsgeschwindigkeit, falls eine spezifische (ionische oder hydrophobe) Wechselwirkung zwischen dem Wirkstoff und den Substituenten stattfindet.

Der theoretische, maximale Substitutionsgrad beträgt 3 (3 Substituenten pro Glukoseeinheit); zuviele Substituenten bewirken jedoch eine sterische Hinderung bei der Komplexbildung. Im Falle kleinerer Substituenten (z.B. $C_{1-3}$ Alkyl, Aryl) wird ein Substitutionsgrad von ≦ 2 bevorzugt, bei größeren Substituen-

ten (z.B. $C_{4-20}$ Alkyl, Acyl und Aryl, Arylalkyl und Silylgruppen), wird ein Substitutionsgrad von ≦ 1 bevorzugt.

Der Wirkstoff kann entweder im Innern der Cyclodextrinhohlräume in Form von Einschlußkomplexen lokalisiert oder von den durch die Quervernetzungen gebildeten Hohlräumen absorbiert oder einfach auf der Oberfläche der Polymerkügelchen adsorbiert sein. Infolge der Aufteilung des Verteilungsgleichgewichts zwischen dem Polymer und dem Lösungsmedium kann der geringere Gehalt an Wirkstoff eine geringere Auflösungsgeschwindigkeit bewirken. Bei Verwendung ionischer Wirkstoffe besteht weiterhin die Möglichkeit, die Freisetzungsgeschwindigkeit durch die Verwendung ionischer Polymere zu steuern. Die Salzbildung kann die Komplexbildung und Absorption fördern. Die lipophilen Cyclodextrinderivate (z.B. Methyl-, Acetyl-, Silylderivate) können mit Wirkstoffen, die große hydrophobe Gruppen enthalten, eine Verringerung der Wirkstofffreisetzung bewirken. Die hydrophoben Wechselwirkungen können weiterhin die Komplexbildung und Absorption fördern. Andererseits kann eine große Zahl funktioneller Gruppen eine sterische Hinderung der Komplexbildung bewirken, wodurch sich ebenfalls eine verzögerte Freisetzungsgeschwindigkeit ergibt.

Die erfindungsgemäß verwendbaren Wirkstoffe können insbesondere aus der Gruppe von Wirkstoffen mit desinfizierender, antiseptischer, antimykotischer, entzündungshemmender, antibiotischer und/oder anästhetischer Wirkung ausgewählt werden, also derjenigen Wirkstoffe, die im allgemeinen auch zur Lokalbehandlung verwendet werden. Unter den Antiseptika sind erfindungsgemäß insbesondere bevorzugt Salicylsäure und ihre Abkömmlinge, Chloramphenicol, Chamazulen, Iod, Phenol, Thymol, Benzoesäure, Benzalkoniumchlorid, Resorcin, Cetylpyridiniumsalze, Anilinfarbstoffe wie Fuchsin, Gentianaviolett, Brilliantgrün, Methylenblau und Acridinderivate. Unter den entzündungshemmenden Wirkstoffen sind bevorzugt Azulen, Hydrocortison, Prednisolon, Depersolon und Triamcinolon. Aus der Gruppe der Lokalanästhetika sind bevorzugt Lodocain, Tetracain, Chlorprocain, Cyclomethycain, Benzocain, Xylocain, Etidocain, Oxycain und Procain. Unter den Antibiotika sind bevorzugt Methycillin, Oxacillin, Semicillin, Pyrazocillin, Streptomycin, Dihydrostreptomycin, Gentamycin, Chloramphenicol, Oxytetracyclin und Biomycin, aus der Gruppe der Antimykotika Nystatin, Amphotericin B, Tolnaftat, Miconazol, Ketoconazol. Erfindungsgemäß kann auch eine Mischung dieser Wirkstoffe verwendet werden, bevorzugt eine Zusammensetzung eines antiseptisch wirkenden und eines entzündungshemmenden Wirkstoffs oder eine Zusammensetzung eines antiseptisch und eines anästhetisch wirkenden Wirkstoffs.

Die EP-84 116 371.0 beschreibt γ-Cyclodextrin-Komplexe mit Nystatin und Amphotericin B. Die Ausgangsverbindung β-Cyclodextrin zeigte bei der Komplexierung dieser Polyen-Antibiotika keine Wirkung. Eine Lösung von Amphotericin B und Nystatin in Wasser kann sowohl in Gegenwart als auch in Abwesenheit von β-Cyclodextrin kaum festgestellt werden, und die zugehörigen UV-Spektren zeigen eine beträchtliche Zerstörung.

Überraschenderweise wurde nunmehr erfindungsgemäß gefunden, daß die β-Cyclodextrinpolymere mit Nystatin und Amphotericin B Komplexe bilden, was durch die UV-Spektren der gelösten Antibiotika nachweisbar war.

Nystatin zeigt ein sehr charakteristisches UV-Spektrum mit 3 scharfen UV-Maxima zwischen 290 und 320 nm (Fig. 1 ), welches auf das Tetraenchromophor zurückzuführen ist.

Der Wechsel im UV-Spektrum zeigt die Zerstörung des konjungierten Chromophorsystems an, von welchem angenommen wird, daß es für die biologische Aktivität verantwortlich ist.

Das Spektrum einer Nystatinprobe in wäßriger Lösung kann nicht ausgewertet werden, denn das Maximum bei etwa 290 nm verschwindet fast völlig, während bei einem niedrigerem Wellenlängenbereich ein neues Maximum auftaucht (Fig. 3 ). Auch mit β-Cyclodextrin komplexiertes Nystatin zeigt das gleiche Verhalten (Fig. 4 ).

Fig. 2 zeigt die UV-Spektren von Nystatin, welches erfindungsgemäß in substituiertem oder unsubstituiertem β-Cyclodextrinpolymeren gelöst ist.

Die Spektren sind identisch mit denen von Nystatin, gelöst in 50% Ethanol (Fig. 1 ) oder in γ-Cyclodextrin. Dies zeigt, daß β-Cyclodextrinpolymere für die Komplexierung von Nystatin geeignet sind. Fig. 2 zeigt weiterhin, daß die β-Cyclodextrinpolymere - im Gegensatz zu den β-Cyclodextrinen - Nystatin stabilisieren, und zwar in ähnlicher Weise wie γ-Cyclodextrin.

Die erfindungsgemäß bevorzugt einsetzbaren Polymere sind unlösliche, vernetzte α-, β- oder γ-Cyclodextrinpolymere und/oder deren Derivate, die in herkömmlicher Weise herstellbar sind, bevorzugt durch Vernetzung mit einer Epoxyverbindung (US 4 274 985/1981). Die ionischen (z.B. Carboxyalkyl-, Alkylamino-, Sulphalkyl-) und nicht-ionischen-(Alkyl-, Arylalkyl-, Acyl-, Alkylthio-, Alkylsulfonyl-, Silyl- ) Derivate werden ebenso in herkömmlicher Weise hergestellt (Jpn. Kokai 59 26,142/1982, 59 150 544/1984, 59 36 549/1984). Der Substitutionsgrad variiert von 0 - 2.

Weiterhin sind erfindungsgemäß insbesondere bevorzugt lipophile Cyclodextrinpolymere einsetzbar, die im Anschluß an Beispiel 22 im Einzelnen beschrieben sind.

Abhängig von den Eigenschaften des aktiven Bestandteils kann eine geeignete Verzögerung der Freisetzung ggf. durch das unmodifizierte Cyclodextrinpolymer erreicht werden. Falls die unmodifizierten Cyclodextrinpolymere nicht wirksam sind, können Polymere mit ionischen oder nicht-ionischen Substituenten verwendet werden. Durch die Auswahl der geeigneten funktionellen Gruppe bzw. Gruppen und durch die Veränderung des Substitutionsgrades kann die gewünschte Freisetzungsgeschwindigkeit erreicht werden.

Die Polymer-Wirkstoff-Zusammensetzungen können erfindungsgemäß hergestellt werden durch a) durch Quellen des Polymers in einer geeigneten Wirkstofflösung, b) durch Mischen des Polymers und des Wirkstoffpulvers in trocknem Zustand und Quellen in einer geeigneten Lösung. Die Suspension wird bevorzugt gerührt oder geschüttelt, falls notwendig filtriert und anschließend getrocknet. Die Lösung zur Auflösung des Wirkstoffs und zum Quellen kann eine wäßrige, eine wäßrig-alkoholische oder eine gepufferte Lösung sein. Das Erzeugnis kann - je nach den Eigenschaften des Wirkstoffs - bei Raumtemperatur oder bei erhöhten Temperaturen getrocknet werden. Das Quellen und Rühren wird bei 0 °C bis 60 °C, bevorzugt bei Raumtemperatur für 0,5 - 24 Stunden, bevorzugt 1 bis 6 Stunden lang, durchgeführt. Falls eine Mischung aus verschiedenen Wirkstoffen gewünscht wird, kann eine herkömmliche Lösung der Wirkstoffe zum Quellen des Polymeren verwendet werden oder die Polymer-Wirkstoff-Zusammensetzungen können entweder im gequollenen oder trockenen Zustand vermischt werden. Der Wirkstoffgehalt im Polymer kann 0,001 - 20 %, bevorzugt 0,5 - 5 Gew.-% betragen.

Die erfindungsgemäßen Polymer-Wirkstoff-Zusammensetzungen sind beispielsweise zur Behandlung infizierter Wunden, Geschwüre, traumatischer Verletzungen und Verbrennungen verwendbar. Diese Zubereitungen weisen eine zweifache Wirkung auf. Das Polymer absorbiert das Wundexudat durch Aufquellen und entfernt die Abbauprodukte, Bakterien und deren Toxine aus der Wundoberfläche, andererseits wird der Wirkstoff stetig aus dem gequollenen Polymer freigesetzt. Derjenige Teil des Wirkstoffs, der nur auf der Polymeroberfläche absorbiert ist, wird sofort nach der Anwendung, der komplexierte Teil dagegen stetig freigesetzt, wodurch eine konstante Wirkstoffkonzentration auf der infizierten Oberfläche erreicht wird. Die Freisetzungsgeschwindigkeit wird wegen des Verteilungsgleichgewichts zwischen dem Polymeren und der Flüssigkeit durch das Volumen des Exudats beeinflußt. Bei Behandlungsbeginn, wenn die wunde Stelle stark näßt, wird eine größere Wirkstoffmenge aus der Polymer-Wirkstoff-Zusammensetzung freigesetzt. Bei fortschreitendem Heilungsprozeß und abnehmender Absonderung vermindert sich gleichfalls die aus dem Polymeren abgegebene Wirkstoffmenge. Dieser Autoregulationsmechanismus wird durch die erfindunsgemäßen Zusammensetzungen erreicht und ist aus dem Stand der Technik nicht bekannt oder nahegelegt.

Die erfindungsgemäßen Polymer-Wirkstoff-Zusammensetzungen können direkt als Wundpuder oder in Form einer Suspension in Glycerin oder einer anderen geeigneten Flüssigkeit in Form einer Paste, Salbe oder dergleichen verwendet werden.

<u>Beispiel 1</u>

2 g Polymere (A : β-Cyclodextrinpolymer (BCDP); B:Diethylenaminoethyl-BCDP (DEAE-BCDP); C: Carboxymethyl-BCDP (CM-BCDP)) wurden in 8 ml einer wäßrigen Ethanollösung (50 %v/v), mit einem Gehalt an 1,25 % Salicylsäure gequollen, zwei Stunden lang geschüttelt, bei Raumtemperatur über Nacht und anschließend bei 105 °C weitere zwei Stunden lang getrocknet. Die Erzeugnisse sind gekennzeichnet durch ihren Gehalt an Salicylsäure und ihre Auflösungseigenschaften.

Der Salicylsäuregehalt wurde bestimmt durch wiederholtes Kochen der 0,25 g Probe in 20 ml Phosphatpufferlösung (pH = 7,2), Filtrierung und anschließender photometrischer Messung der Konzentration im abgekühlten Filtrat bei $\lambda$ = 296 nm. Dieses Verfahren wurde vier Mal wiederholt und die Summe der Ergebnisse errechnet.

Die Auflösungsgeschwindigkeit wurde durch Rühren von 0,2 g der Probe mit einem Magnetrührer in 20 ml destilliertem Wasser oder in einer Phosphatpufferlösung von pH = 7,2 bei 37 °C bestimmt. Proben aus dem Lösungsüberstand wurden zu bestimmten Zeiten (im Bereich von 1 - 60 Min.) entnommen und die Konzentration spektrophotometrisch bestimmt. Die Erzeugnisse sind gekennzeichnet durch die gelöste Menge in 60 Min., ausgedrückt in Prozent der Gesamtaufnahme an aktivem Bestandteil.

| Beschreibung des β-CD-Polymers | | | Eigenschaften der Produkte | | |
|---|---|---|---|---|---|
| ionische Gruppe | Gehalt an ionischer Gruppe (meq/g) | Quellung (ml/g) | Salicylsäure Gehalt (%) | gelöste Menge in Wasser in 60 Min. (%) | gelöste Mengein Puffer in 60 Min. (%) |
| - | - | 5,0 | 4,3 | 54 | 81 |
| DEAE | 0,43 | 4,0 | 4,1 | 18 | 61 |
| CM | 0,62 | 3,6 | 4,4 | 58 | 99 |

Beispiel 2

1,2 g Ethacridinlactat werden in 90 ml 50% (v/v) Ethanol gelöst. 6 ml dieser Lösung werden zu 1,5 g β-Cyclodextrinpolymer zum Quellen gegeben. Die Suspension wird 1 Stunde lang geschüttelt, an der Luft zum Trocknen stehengelassen und anschließend bei 105 ° C zwei Stunden lang getrocknet.

Der Gehalt an Ethacridinlactat wird gem. Beispiel 1 bestimmt mit der Ausnahme, daß die Probe in einer Lösung aus etwa 0,1 g Benzoesäure in 50 % (v/v) Ethanol gekocht und anschließend spektrophotometrisch bei λ = 370 nm gemessen wird. Die Auflösungsgeschwindigkeit wird, wie im Beispiel 1 beschrieben, bestimmt.

| Beschreibung des β-CD-Polymers | | | Eigenschaften der Produkte | |
|---|---|---|---|---|
| ionische Gruppe | Gehalt an ionischer Gruppe (meq/g) | Quellung (ml/g) | Ethacridinlactat-Gehalt (%) | gelöste Menge in Wasser in 60 Min. (%) |
| - | - | 5,0 | 5,0 | 86 |
| CM | 0,04 | 5,0 | 5,2 | 70 |
| CM | 0,07 | 3,3 | 5,3 | 72 |
| CM | 0,09 | 3,7 | 4,9 | 61 |
| CM | 0,11 | 4,7 | 5,3 | 50 |
| CM | 0,17 | 4,3 | 5,2 | 27 |
| CM | 0,22 | 4,4 | 5,4 | 20 |
| CM | 0,35 | 4,8 | 5,4 | 7,7 |
| CM | 0,62 | 3,6 | 5,2 | 3,5 |
| CM | 0,73 | 3,1 | 4,7 | 1,5 |

Beispiel 3

Ethacridinlactat (EAL)-Zubereitungen mit verschiedenen EAL/CD-Verhältnissen werden wie folgt hergestellt :

0,4 g EAL wurden in 15 ml 50 % (v/v) wäßrigem Ethanol gelöst. Diese Stammlösung wurde in geeigneter Weise verdünnt und von jeder dieser Lösungen wurden 4 ml zu 1 g nicht-modifiziertem β-Cyclodextrinpolymer (quellen : 5,0 ml/g) gegeben, die Suspension wurde geschüttelt und anschließend getrocknet. Der Gehalt an EAL und die Auflösung wurden gemäß Beispiel 2 bestimmt.

| Ethacridinlactat Gehalt (%) | EAL/CD molares Verhältnis | gelöste Menge EAL in Wasser in 60 Min. (%) |
|---|---|---|
| 9,6 | 0,64 | 94,3 |
| 5,3 | 0,35 | 89,4 |
| 2,6 | 0,17 | 70,0 |
| 1,1 | 0,07 | 23,4 |
| 0,53 | 0,04 | 4,2 |
| 0,26 | 0,02 | 2,3 |

Beispiel 4

0,24 g Methylenblau werden in 90 ml 50 % (v/v) Ethanol gelöst. 20 ml dieser Lösung werden zu 5 g β-Cyclodextrinpolymer zum Quellen gegeben. Die Suspension wird 2 Stunden lang geschüttelt, an der Luft zum Trocken stehengelassen und anschließend bei 105 °C getrocknet.
Die Produkte werden wie im Beispiel 2 beschrieben bestimmt, wobei die spektrophotometrischen Messungen bei λ = 661 nm durchgeführt werden.

| Beschreibung des β-CD-Polymers | | | Eigenschaften der Produkte | |
|---|---|---|---|---|
| ionische Gruppe | Gehalt an ionischer Gruppe (meq/g) | Quellung (ml/g) | Methylenblau Gehalt (%) | gelöste Menge in Wasser in 60 Min. (%) |
| - | - | 5,0 | 1,2 | 34 |
| CM | 0,06 | 4,8 | 1,3 | 32 |
| CM | 0,62 | 3,6 | 1,2 | 9,2 |

Beispiel 5

Methylenblau-Zubereitungen mit verschiedenen Methylenblau/CD-Verhältnissen werden in folgender Weise hergestellt :
0,8 g Methylenblau werden in 30 ml 50 % (v/v) an wäßrigem Ethanol gelöst. Diese Stammlösung wird geeigneterweise verdünnt und von jeder dieser Verdünnungen werden 8 ml zu 2 g nicht-modifiziertem β-Cyclodextrinpolymer gegeben (quellen : 5,0 ml/g).
Der Methylenblau-Gehalt und die Auflösungsgeschwindigkeit wurden wie im Beispiel 4 beschrieben bestimmt.

| Methylenblau Gehalt (%) | Methylenblau/CD molares Verhältnis | Gelöstes Methylenblau in Wasser in 60 Min (%) |
|---|---|---|
| 9,6 | 0,58 | 99,7 |
| 5,1 | 0,31 | 68,8 |
| 2,6 | 0,16 | 72,6 |
| 1,3 | 0,07 | 34,6 |
| 0,53 | 0,03 | 10,0 |
| 0,26 | 0,02 | 5,4 |

Beispiel 6

Zubereitungen mit einer kontrollierten Freisetzung und einem Gehalt von 2 Gew.-% Triphenylmethan-Farbstoffen wurden wie folgt hergestellt :
0,105 g an Triphenylmethan-Farbstoff (Gentianaviolett, Brilliantgrün, basisches Fuchsin oder Fuchsinsäure) wurden in 20 ml 50 % (v/v) Ethanol gelöst.
8 ml dieser Lösung wurden zu 2 g β-Cyclodextrinpolymer (nicht-modifiziertes Polymer und Polymer mit

einem Gehalt an 0,22 ml/g Carboxymethyl, 4,0 ml/g Quellung). Die Suspension wurde 1,5 Stunden lang geschüttelt, an der Luft und anschließend bei 105 °C getrocknet.

Der Gehalt an aktiven Bestandteil wurde auf der Basis des Ansatzes der Zubereitung bestimmt.

Die gelöste Menge wurde wie im Beispiel 2 angegeben spektrophotometrisch bei λ = 580 nm (Gentianaviolett), λ = 628 nm (Brilliantgrün), λ = 553 nm (basisches Fuchsin) und λ = 550 nm (Fuchsinsäure) bestimmt.

| Färbemittel | Carboxymethylgruppengehalt (meq/g) | Quellung (ml/g) | gelöste Menge in Wasser in 60 Min (%) |
|---|---|---|---|
| Gentianaviolett | -<br>0,22 | 5,0<br>4,0 | 6<br>0,25 |
| Brilliantgrün | -<br>0,22 | 5,0<br>4,0 | 23<br>1,7 |
| basisches Fuchsin | -<br>0,22 | 5,0<br>4,0 | 7,5<br>0,9 |
| Fuchsinsäure | -<br>0,22 | 5,0<br>4,0 | 17<br>1,2 |

Beispiel 7

Zubereitungen mit kontrollierter Freisetzung, die Eosin enthalten, werden durch Lösen von 0,025 g oder 0,105 g Eosin in 20 ml 50% (v/v) Ethanol hergestellt. 4 ml dieser Lösung werden zu 1 g β-Cyclodextrinpolymer gegeben, 2 Stunden lang geschüttelt und bei 105 °C getrocknet.

Der Eosin-Gehalt wird durch wiederholtes Kochen in 1:1 Ethanol-Wasser, enthaltend 5 Gew.-% $Na_2CO_3$ , bestimmt und die Eosin-Konzentrationen spektrophotometrisch bei λ = 525 nm bestimmt.
Die Auflösungsgeschwindigkeit wird in Wasser oder in einer Pufferlösung von pH = 7,5 , wie in Beispiel 1 beschrieben, bestimmt.

| Beschreibung des β-CD-Polymers | | Eigenschaften des Produkts | | |
|---|---|---|---|---|
| funktionelle Gruppe | funktioneller Gruppengehalt (meq/g) | Eosin Gehalt | Gelöst in 60 Min in Wasser | in Puffer |
| | | | (%) | (%) |
| - | - | 0,5 | 100 | |
| Silyl | 0,30 | 0,5 | 75 | |
| Acetyl | 4,2 | 0,5 | 156 | |
| - | - | 1,9 | 100 | |
| CM | 0,62 | 1,9 | 63 | |
| DEAE | 0,43 | 1,9 | 0,01 | 3 |

Beispiel 8

0,6 g Nystatin werden in 45,0 ml 50 % (v/v) Ethanol gelöst und 12 ml dieser Lösung zu 3 g β-Cyclodextrinpolymer gegeben. Die Suspension wird geschüttelt und anschließend getrocknet. Die Auflösungsgeschwindigkeit wird wie im Beispiel 1 spektrophotometrisch bei λ = 306 nm gemessen.

| Beschreibung des β-CD-Polymers | | | Eigenschaften des Produktes | |
|---|---|---|---|---|
| funktionelle Gruppe | funktioneller Gruppengehalt (meq/g) | Quellung (ml/g) | Nystatin Gehalt (berechnet %) | gelöst in Wasser in 60 Min bei 37 °C (%) |
| - | - | 5,0 | 5,1 | 22,4 |
| - | - | 3,8 | 5,1 | 22,8 |
| DEAE | 0,63 | 5,0 | 5,1 | 10,4 |
| Methyl | 0,73 | 4,0 | 5,1 | 46,6 |

*vernetzt mit Butylenglycol-bis(epoxypropyl)ether

Beispiel 9

Lidocain enthaltende Produkte werden wie folgt zubereitet : 60 ml Lidocain werden in 22,5 ml 50 % (v/v) Ethanol gelöst. 4 ml dieser Lösung werden zu 1 g des Polymeren zum Quellen gegeben.
Die Suspension wird für 2 Stunden geschüttelt, an der Luft übernacht getrocknet und anschließend 2 Stunden lang bei 105 °C getrocknet.
Die Produkte werden wie im Beispiel 1 beschrieben charakterisiert. Die Extinktion bei λ max = 262 ± 1 nm wurde zur Bestimmung des gelösten Lidocains verwendet.

| Beschreibung des Polymers | | | Eigenschaften des Produktes | |
|---|---|---|---|---|
| funktionelle Gruppe | funktioneller Gruppengehalt (meq/g) | Quellung (ml/g) | Lidocain Gehalt (berechnet %) | gelöst in Wasser in 60 Min (%) |
| - | - | 5,0 | 1 | 92 |
| Acetyl | 4,2 | 2,0 | 1 | 77 |

Beispiel 10

Triamcinolonacetonid-enthaltende Produkte:
60 mg Triamcinolonacetonid werden in 22,5 ml 50% (v/v) Ethanol gelöst. 4 ml dieser Lösung werden zu 1 g des Polymeren zum Quellen gegeben. Die Suspension wird für 2 Stunden geschüttelt, an der Luft zum Trocknen stehengelassen und 2 Stunden lang bei 105 °C getrocknet. Eine Polymerprobe mit einem Wirkstoffgehalt von 5 % wird durch Zugabe von 25 mg des gelösten Wirkstoffs in 3 ml Ethanol zu 1 g Polymer hergestellt. Die Extinktion bei λ max 242 ± 1 nm wurde zur Bestimmung des gelösten Triamcinolonacetonids verwendet.

| Beschreibung des Polymers | | | Eigenschaften der Produkte | |
|---|---|---|---|---|
| ionische Gruppe | Gehalt an ionischer Gruppe (meq/g) | Quellung (ml/g) | Triamcinolon acetonid Gehalt (%) | gelöst in Wasser in 60 Min. (%) |
| -* | - | 3,8 | 1 | 48,0 |
| DEAE* | 0,83 | 5,0 | 1 | 24,3 |
| Methyl | 0,73 | 4,0 | 1 | 26,1 |
| Acetyl | 4,2 | 2,0 | 1 | 11,4 |
| Acetyl | 4,2 | 2,0 | 5 | 20,3 |

* vernetzt mit Butylenglycol-bis(epoxy-propylether).

Beispiel 11

1 Gew.-% Chlorocid-enthaltende Produkte werden wie folgt hergestellt .
60 mg Chlorocid werden in 22,5 ml 50 % (v/v) Ethanol gelöst. 4 ml dieser Lösung werden zu 1 g des

Polymeren zum Quellen gegeben. Die Suspension wird 2 Stunden lang geschüttelt, an der Luft über Nacht zum Trocknen stehengelassen und anschließend bei 105 °C 2 Stunden lang getrocknet. Die Extinktion bei λ max = 276 ± 1 nm wird zur Bestimmung des gelösten Wirkstoffs verwendet.

| Beschreibung des Polymers | | | Eigenschaften des Produktes | |
|---|---|---|---|---|
| funktionelle Gruppe | funktioneller Gruppengehalt (meq/g) | Quellung (ml/g) | Chlorocid Gehalt % | gelöst in Wasser in 60 Min |
| - | - | 5,0 | | 86,7 |
| Methyl | 0,32 | 5,4 | | 75,0 |
| Butyl** | 0,30 | 4,0 | | 71,0 |

**α-Cyclodextrinpolymer

Beispiel 12

Amphotericin B (AMB) enthaltende Produkte werden durch Lösen von 20 mg AMB in 0,2 ml DMSO, verdünnt auf 20 ml mit 50 % (v/v) Ethanol, hergestellt. 5 ml dieser Lösung werden zu 1 g β-CD-Polymer zum Quellen gegeben. Die Suspension wird für 2 Stunden geschüttelt, an der Luft lichtgeschützt zum Trocknen stehengelassen und anschließend bei 60 °C 1 Stunde lang getrocknet. Die Extinktion bei 382 ± 1 nm wird zur Bestimmung des gelösten AMB verwendet.

| Beschreibung des Polymers | | | Eigenschaften des Produktes | |
|---|---|---|---|---|
| funktionelle Gruppe | funktioneller Gruppengehalt (meq/g) | Quellung (ml/g) | AMB Gehalt (berechnet %) | gelöst in Wasser in 60 Min (%) |
| -* | - | 3,8 | 0,5 | 3,2 |

*vernetzt mit Butylenglycol-bis(epoxypropyl)ether.

Beispiel 13

Eine Wundpuderzusammensetzung enthaltend 0,5 % Hydrocortison, als entzündungshemmenden Wirkstoff und 1 % Tetracain als Lokalanestetikum wird wie folgt hergestellt:
0,5 g Hydrocortison werden in 380 ml 50 % (v/v) wäßrigem Ethanol gelöst und in 100 g Gamma-Cyclodextrinpolymer (gequollen: 4,2 ml/g) werden in dieser Lösung suspendiert und 6 Std. lang gerührt. Das Lösungsmittel wird durch Trocknen bei 50 °C unter Vakuum verdampft. 1 g Tetracain wird in 200 ml 75 % (v/v) wäßrigem Ethanol gelöst. Das getrocknete Produkt wird in dieser Lösung suspendiert und 2 Std. lang gerührt. Das Lösungsmittel wird bei 50 °C unter Vakuum verdampft. Zu diesem Produkt wird 0,1 g Magnesiumstearat gemischt.

Dieser erfindungsgemäße Wundpuder zeigt eine langsame Freisetzung der Wirkstoffe Hydrocortison und Tetracain.

Beispiel 14

Eine Wundpuderzusammensetzung, enthaltend 0,4 % Prednisolon, als entzündungshemmendes Mittel und 0,7 % Thymol als Antiseptikum wird wie folgt hergestellt:
0,7 g Thymol und 0,4 g Prednisolon werden in 430 ml 40 % (v/v) wäßrigem Ethanol gelöst, und in diese

Lösung werden 100 g γ-Cyclodextrinpolymer suspendiert und 2 Std. lang gerührt. Das Lösungsmittel wird bei 50°C unter Vakuum abgedampft. Zu diesem Erzeugnis wird 0,1 g Magnesiumstearat gemischt.

Dieser erfindungsgemäße Wundpuder zeigt eine langsame Freisetzungsgeschwindigkeit beider Wirkstoffe Prednisolon und Thymol.

Beispiel 15

Eine Wundpuderzusammensetzung, enthaltend 0,65 % Ethacridinlactat als Antiseptikum und 3,8 % Nystatin as Antimycotikum wird wie folgt hergestellt.

1 g Produkt, hergestellt wie im Beispiel 3 beschrieben, enthaltend 2,6 % Ethacridinlactat mit unsubstituiertem β-Cyclodextrinpolymer und 3 g Produkt, enthaltend 5,1 % Nystatin, hergestellt wie im Beispiel 8 beschrieben, mit einem Methyl-β-Cyclodextrinpolymer, werden zusammen vermischt, um einen Wundpuder zu ergeben, der die beiden Wirkstoffe Ethacridinlactat und Nystatin nur langsam freisetzt.

Beispiel 16

Eine 0,25 % Amphotericin und 2,5 % Triamcinolonacetonid enthaltende Wundpuderzusammensetzung wird wie folgt hergestellt:

1 g Produkt, hergestellt wie in Beispiel 12 beschrieben, enthaltend 015 % Amphothericin mit unsubstituiertem β-Cyclodextrinpolymer, und 1 g Produkt, enthaltend 5 % Triamcinolonacetonid, hergestellt wie im Beispiel 10 beschrieben, mit Acetyl-β-Cyclodextrinpolymer, werden zu einem Wundpuder vermischt, der beide Wirkstoffe langsam freisetzt.

Beispiel 17

Eine 1 % Lidocain als Lokalanestetikum und 4% Tolnaphtat als Antimycotikum enthaltende Wundpuderzusammensetzung wird wie folgt hergestellt:

1 g Produkt, hergestellt wie im Beispiel 9 beschrieben, enthaltend 1 % Lidocain mit Acetyl-β-Cyclodextrinpolymer, wird in 4 ml einer Lösung von 40 mg Tolnaphtat in 85 % (v/v) wäßrigem Ethanol suspendiert. Das Lösungsmittel wird bei 50°C unter Vakuum abgedampft, um einen Wundpuder zu ergeben, der beide Wirkstoffe nur langsam freisetzt.

Beispiel 18

Eine 5,1 % Nystatin als Antimycotikum und 1 % Streptomycin als Antibiotikum enthaltende Wundpuderzusammensetzung wird wie folgt hergestellt:

3 g Produkt, hergestellt wie im Beispiel 8 beschrieben, enthaltend 5,1 % Nystatin komplexiert mit Diethylaminoethyl-β-Cyclodextrinpolymer, wird in einer 12 ml Lösung aus 40 mg Streptomycin in 85 % (v/v) wäßrigem Ethanol suspendiert. Das Lösungsmittel wird bei 50°C unter Vakuum abgedampft, um einen Wundpuder mit einer langsamen Freisetzung beider Wirkstoffe zu ergeben.

Beispiel 19

Eine 2 % Gentianaviolet als Antiseptikum und 0,5 % Chlorprocain als Lokalanestetikum enthaltende Wundpuderzusammensetzung wird wie folgt hergestellt:

2 g Produkt, hergestellt wie im Beispiel 6 beschrieben, enthaltend 2 % Gentianaviolet mit Carboxymethyl-β-Cyclodextrinpolymer, wird in einer 7 ml Lösung aus 10 mg Chlorprocain in 85 % (v/v) wäßrigem Ethanol suspendiert. Das Lösungsmittel wird unter Vakuum bei 50°C verdampft, um einen Wundpuder zu ergeben, der beide Wirkstoffe langsam freisetzt.

Beispiel 20

Ein 0,5 % Eosin als Antiseptikum und 0,5 % Oxacillin als Antibiotikum enthaltender Wundpuder wird wie folgt hergestellt:

1 g Produkt, hergestellt wie im Beispiel 7 beschrieben, enthaltend 0,5 % Eosin mit Acetyl-β-Cyclodextrinpolymer, wird in einer 3,5 ml Lösung aus 5 mg Oxacillin in 85 % (v/v) wäßrigem Ethanol suspendiert. Das Lösungsmittel wird bei 50°C unter Vakuum abgedampft, um einen Wundpuder mit langsamer Freisetzungsgeschwindigkeit zu erhalten.

Beispiel 21

Eine 0,3 % Oxytetracyclin als Antibiotikum und 0,7 % Piroxicam als entzündungshemmendes Mittel enthaltende Wundpuderzusammensetzung wird wie folgt hergestellt:
0,3 g Oxytetracyclin werden in 360 ml 50 % (v/v) wäßrigem Ethanol aufgelöst, und 100 g γ-Cyclodextrinpolymer wird in dieser Lösung suspendiert und 3 Std. lang gerührt. Das Lösungsmittel wird bei 50 °C unter Vakuum abgedampft. 0,7 g Piroxicam wird in 200 ml 85 % (v/v) wäßrigem Ethanol aufgelöst. Das getrocknete Produkt wird in dieser Lösung suspendiert und 2 Std. lang gerührt. Das Lösungsmittel wird unter Vakuum bei 50 °C abgedampft. Zu diesem Produkt wird 0,1 g Magnesiumstearat gemischt.

Der auf diese Weise erhaltene Wundpuder zeigt eine langsame Freisetzunggeschwindigkeit beider Wirkstoffe.

Beispiel 22

Eine 1,2 % Benzocain als Lokalanestetikum und 0,5 % Gentamicin als Antibiotikum enthaltende Wundpuderzusammensetzung wird wie folgt hergestellt:
1,2 g Benzocain wird in 370 ml 50 % (v/v) wäßrigem Ethanol aufgelöst, und 100 g γ-Cyclodextrinpolymer wird in dieser Lösung suspendiert und 2 Std. lang gerührt. Das Lösungsmittel wird unter Vakuum bei 50 °C abgedampft. 0,5 g Gentamicin werden in 200 ml 80 % (v/v) wäßrigem Ethanol aufgelöst. Das getrocknete Produkt wird in dieser Lösung suspendiert und 2 Std. lang gerührt. Das Lösungsmittel wird bei 50 °C unter Vakuum abgedampft. Zu diesem Produkt wird 0,2 g Aerosil gemischt.

Der so erhaltene Wundpuder zeigt eine langsame Freisetzung beider Wirkstoffe.

Im folgenden werden lipophile Cyclodextrinpolymere offenbart, die in den erfindungsgemäßen Einschlußkomplexen bevorzugt einsetzbar sind.

Es ist bekannt, daß die Hydroxylgruppen auf den Cyclodextrinmolekülen derivatisierbar sind. Die Reaktivität der Hydroxylgruppen an verschiedenen Positionen sowie die Verfahren zur Derivatisierung sind in verschiedenen Artikeln beschrieben (Liptak, A., Fugedi P., Szurmai, Z., Imre, J., Nanasi, P., Szejtli, J.: Proc. 1. Intern. Symp. für Cyclodextrine, Reidel, Dordrecht (1982) 275, Croft, A., Bartsch, R.A.: Tetrahedron 39 (1983) 1417).

Cyclodextrinderivate mit einem hohen Molekulargewicht, die mehrere Cyclodextrinringe in einem Molekül enthalten, sind durch Vernetzung mit bi- oder polyfunktionalen Reagenzien (z.B. Diepoxiden, Diisocyanaten), die mit den Hydroxylgruppen reagieren können, herstellbar (Zsadon, B., Fenyvesi,E.: Proc. 1. Intern. Symp. für Cyclodextrine, Reidel, Dordrecht (1982) 327).

Diese sogenannten Cyclodextrinpolymere können von einem niedrigeren Molekulargewicht sein und sind dann wasserlöslich oder sie weisen ein höheres Molekulargewicht auf und sind dann in Wasser quellfähig. Nicht alle Hydroxylgruppen sind mit Querverbindungen besetzt; die verbleibenden Hydroxylgruppen können mit anderen funktionellen Gruppen substituiert sein. Derart hergestellte Cyclodextrinpolymere sind für verschiedene Zwecke verwendbar.

Mit ionischen Gruppen modifizierte Cyclodextrinpolymere sind bekannt. Die wasserlöslichen Polymere mit ionischen Gruppen (Ung. Patent 191 101 (1986), US-Patent 4 545 152) werden bevorzugt in der Fotoindustrie verwendet (Szucs, H., Kiss, I.: Inf. Rec. mater, 14 (1986) 383 und 16 (1988) 439). Die Polymere mit den ionischen Gruppen, die im Wasser quellfähig sind, werden als spezifische Ionenaustauscher mit Komplexbildungsfähigkeit verwendet. Cyclodextrine mit Carboxymethyl- und Sulfalkylgruppen besitzen Kationenaustausch-, die mit Diallylaminogruppen substituierten Anionenaustauscheigenschaften (Jap. Patente 59 26142, 59 150549, 59 36549, 1984). Cyclodextrinpolymere mit Aminoalkylaminogruppen sind zur Enzymimmobilisierung verwendbar (Jap. Patent 60248729, 1986). N-Methyl-dihydronicotinamid-β-cyclodextrinpolymer ist als künstliches Enzym patentiert ( JP-62275102, 1987 ).

Es ist eine Aufgabe der Erfindung, neue Cyclodextrinpolymerderivate mit lipophilen Eigenschaften herzustellen.

Diese Aufgabe wird erfindungsgemäß durch lipophile Cyclodextrinderivate der folgenden allgemeinen Formel gelöst:

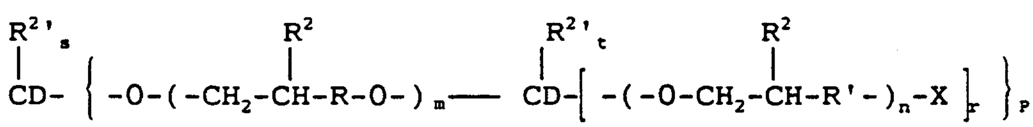

wobei
CD aus einem α-, β- und/oder gamma-Cyclodextrinmolekül durch Abspaltung von p+s- oder 1+t+r-Hydroxylgruppen stammt, R und R'unabhängig voneinander aus der folgenden Gruppe ausgewählt werden : $-CH_2-$ , $-CH0H-CH_2-$ , $-CH_2-0-(CH_2)_2-0-CH_2-CH0H-CH_2-$ , $-CH_2-0-CH_2-CHOH-CH_2-$ or $-CH_2-0-(CH_2)_4-0-CH_2-CHOH-CH_2-$
X $OR^1$ oder $R^2$ bedeutet, wobei
$R^1$ ein Wasserstoffatom oder - falls r nicht 0 ist - eine Gruppe darstellt, welche durch die Abspaltung eines Wasserstoffatoms aus einer Hydroxylgruppe aus einem α-, β- oder γ-Cyclodextrinmolekül erhalten wird.
$R^2$ gleiche oder verschiedene Bedeutungen aufweist, nämlich $0R^3$ oder eine Alkylthio-, Alkylsulfonyl-, Alkylsulfinyl- oder Aminoalkylgruppe mit 1-20 Kohlenstoffatomen, bevorzugt mit 1 bis 6 C-Atomen, oder eine Gruppe darstellt, welche durch die Abspaltung eines Wasserstoffatoms aus einer Hydroxylgruppe aus einem α-, β- oder γ-Cyclodextrinmolekül erhalten wird,
$R^{2'}$ der Bedeutung von $R^2$ entspricht, jedoch keine der Gruppen darstellt, die aus den Cyclodextrinmolekülen erhalten wurden, welche durch die Abspaltung eines Wasserstoffatoms aus einer Hydroxylgruppe aus einem α-, β- oder γ-Cyclodextrinmolekül erhalten werden,
$R^3$ eine Alkyl-Gruppe mit 1 - 20 C-Atomen, bevorzugt mit 1 bis 6 C-Atomen, oder eine Aralkyl-oder Alkylarylgruppe darstellt, wobei die Arylgruppe aus einer aromatischen oder heteroaromatischen Verbindung abstammt, oder $R_3$ eine $C_1$ - $C_7$-Acylgruppe oder eine unsubstituierte oder $C_1$ - $C_5$-mono-, di- oder trisubstituierte Silylgruppe darstellt.
m und n unabhängig voneinander ein ganzzahliges von 1 - 10 sind,
r ein ganzzahliges von 0 - 23 ist,
p ein ganzzahliges von 0 - 24 ist,
s und t unabhängig voneinander ein ganzzahliges von 0 - 7 sind,
m, n, r und t, einschließlich in den Seitenketten, innerhalb einer Einheit je unterschiedlich sein können,
und, falls CD eine Gruppe ist, die aus einem α-Cyclodextrin stammt, $p+s<18$ und $r+t<17$ sind,
und, falls CD eine Gruppe ist, die aus einem β-Cyclodextrin stammt, $p+s<21$ und $r+t<20$ sind,
und, falls CD eine Gruppe ist, die aus einem gamma-Cyclodextrin stammt, $p+s<24$ und $r+t<23$ sind.

Die Alkylgruppen umfassen sowohl n-Alkylgruppen als auch ihre Isomere, bevorzugt $C_1$ - $C_{20}$-Alkylgruppen, insbesondere bevorzugt $C_1$ - $C_{10}$-Alkylgruppen.

Die Arylgruppen umfassen insbesondere Phenyl-, Naphthyl-, Biphenyl-, Anthracyl-Gruppen.

Die Alkylarylgruppen umfassen insbesondere Tolyl-, Xylyl-, Ethylphenyl-, Propylphenyl-, tertiäre Butylphenyl-Gruppen.

Die Aralkylgruppen umfassen insbesondere Benzyl-, Phenethyl-, Benzhydryl- und Trityl-Gruppen.

Die Heteroarylgruppen umfassen insbesondere Piridyl-, Picolyl-, Bipyridyl- und Collidyl-Gruppen.

Die Acylgruppen umfassen insbesondere Formyl-, Acetyl-, Propionyl-, Butyryl-, Isobutyryl-, Valeryl-, Isovaleryl-, Stearyl-, Oleyl- und Palmitoyl-Gruppen.

Die S-enthaltenden Gruppen umfassen insbesondere Methylsulphonyl-, Ethylsulphonyl-, Hexylsulphonyl-, Dodecylsulphonyl-, Methylthio-, Butylthio-, Hexylthio-Gruppen.

Die N-enthaltenden Gruppen umfassen insbesondere Amino-, Methylamino-, Ethylamino-, Propylamino-, Hexylamino-, Dodecylamino- und Imidazoyl-Gruppen.

Die Silylgruppen umfassen insbesondere Trimethylsilyl- und tertiäre Butyldimethylsilyl-Gruppen.

Das Cyclodextrin ist bevorzugt ein β-Cyclodextrinmolekül.

Weiterhin bevorzugt sind die erfindungsgemäßen lipophilen Cyclodextrinpolymere,
dadurch gekennzeichnet,
daß m und n unabhängig voneinander ein Ganzzahliges von 3 bis 7 sind und/oder r ein Ganzzahliges von 5 bis 17 oder von 8 bis 14 ist, und/oder b ein Ganzzahliges von 5 bis 17 oder von 8 bis 14 ist, und/oder s und t unabhängig voneinander ein Ganzzahliges von 2 bis 5 sind und/oder m, n, r und t, einschließlich in den Seitenketten, innerhalb einer Einheit je unterschiedlich sein können, und, falls CD eine Gruppe ist, die aus einem α-Cyclodextrin stammt, $p+s<13$ und $r+t<14$ sind oder $p+s<10$ und $r+t<10$ sind, und, falls CD eine Gruppe ist, die aus einem β-Cyclodextrin stammt, $p+s<17$ und $r+t<16$ sind und/oder $p+s<10$ und $r+t<9$ und, falls CD eine Gruppe ist, die aus einem gamma-Cyclodextrin stammt, $p+s<20$ und $r+t<19$ sind und/oder $p+s<14$ und $r+t<13$ sind.

Diese lipophilen Cyclodextrinpolymere sind in Lösungsmitteln mit einer niedrigen Dielektrizitätskonstante löslich oder quellbar.

Die lipophilen Cyclodextrinpolymere sind erfindungsgemäß durch zwei Verfahren herstellbar :

a) Durch Vernetzung der geeigneten Cyclodextrinderivate oder
b) durch Derivatisierung der Cyclodextrinpolymere.

a) Geeignete Cyclodextrinderivate sind Alkylderivate, bevorzugt Dimethyl-, Hydroxypropyl- und/oder Palmityl-Derivate, Acylderivate, z.B. Acetyl-, Benzoyl-, Succinyl-, Lauryl- und/oder Palmitoylderviate, Alkylthioderivate, z.B. Methylthioderivate, Alkylsulfonylderivate, z.B. Propylsulfonylderivate, Alkylsulfinylderivate, z.B. Ethylsulfinylderivate und Dodecylsulfinylderivate, Desoxyaminoalkylderivate, z.B. Aminoethylderivate, Silylderivate, z.b. Dimethylsilyl-, Trimethylsilyl- und Tert. Butyl-dimethylsilylderivate, wobei der Substitutionsgrad im Bereich von 0,1 - 2,5 , bevorzugt zwischen 0,5-2,0, liegt.

Der Substitutionsgrad (D.S.) bedeutet die durchschnittliche Zahl an Substituenten auf einer Glucoseeinheit.

Der Substituent auf dem Polymer kann, wenn das erfindungsgemäße Cyclodextrinpolymer durch Derivatisierung des bereits polymerisierten Cyclodextrins hergestellt wird, nicht nur auf den Glucoseeinheiten des Cyclodextrinrings lokalisiert sein, sondern auch auf den verbindenden Glycerylbrücken. Es ist deshalb besser, das Produkt durch die Menge an Substituent in einem Gramm Produkt ausgedrückt in Mol. (meq = mole equivalent) zu charakterisieren.

D.S. = 1 entspricht 3 mMol/g oder 3 meq/g.

Das Produkt mit der größten Substituentenmenge in dieser Patentanmeldung ist das Acetyl-BCD mit 4,2 mMol/g (D.S. = 1,4) Acetylgehalt.

Die erfindungsgemäßen Verbindungen können durch bekannte Verfahren hergestellt werden. Durch die Umsetzung mit Epichlorhydrin, Diepoxiden oder anderen bifunktionellen Reagenzien können die Polymerderivate der lipophilen Ausgangsverbindungen hergestellt werden.

b) Cyclodextrinpolymere zur Herstellung ihrer lipophilen Derivate sind erfindungsgemäß lösliche oder quellfähige Produkte, die durch Polykondensation aus $\alpha$-, $\beta$- und/oder gamma-Cyclodextrin hergestellt werden, bevorzugt durch Vernetzung mit Epichlorhydrin oder Diepoxiden. Diese Polymere können durch solche chemischen Reaktionen modifiziert werden, die zur Derivatisierung der Cyclodextrine selbst verwendet werden.

Die Reaktionsbedingungen müssen gemäß den Eigenschaften des Cyclodextrinpolymers ausgewählt werden, d.h. das Reaktionsmedium muß so gewählt werden, daß sich das Polymer entweder löst oder daß es quillt, wobei die Polymerstruktur nicht zerstört werden darf. Alkalische wäßrige Lösungen, N,N-Dimethylformamid, Dimethylsulfoxid und Pyridin sind hierbei bevorzugt.

Erfindungsgemäß werden die Cylcodextrinderivate bevorzugt vernetzt, um ein Polymer herzustellen:

1.a) Das Cyclodextrinderivat wird in einer alkalischen wäßrigen Lösung gelöst oder suspendiert. Die Cyclodextrinkonzentration in der Lösung ist im Bereich von 20 - 50 Gew.-%, bevorzugt 30 - 40 Gew.-%, wenn das gewünschte Produkt löslich ist und im Bereich von 40 - 50 Gew.-%, wenn es ein quellfähiges Polymer ist. Die Alkali- oder Erdalkalihydroxydkonzentration, bevorzugt NaOH oder KOH, ist 2 - 8 Gew.-%, wenn ein Diepoxid zur Vernetzung verwendet wird. Wenn Epichlorhydrin als Vernetzungsmittel verwendet wird, ist das molare Verhältnis von Alkalihydroxyd zu Epichlorhydrin 0,8 - 1,2 : 1, bevorzugt 1 : 1. Das Vernetzungsmittel (Epichlorhydrin, 1,2,9,10-Diepoxy-4,7-dioxadecan, 1,2,11,12-Diepoxy-4,9-dioxadodecan) wird tropfenweise unter Rühren bei 20 - 80 °C zugegeben. Das Molverhältnis des Vernetzungsmittels zum Cyclodextrinderivat beträgt 5 - 20 : 1, bevorzugt 10 - 15 : 1 . Die Reaktionsmischung wird für weitere 1 - 4 Stunden gerührt und anschließend abkühlen gelassen. Falls das Produkt ein lösliches Polymer ist, wird es durch Dialyse oder Chromatographie gereinigt und bevorzugt durch Lyophilisation getrocknet. Falls das Produkt ein quellfähiges Polymer ist, wird es mit Wasser bis zur Neutralität gewaschen, mit wäßrigem Aceton bei zunehmender Acetonkonzentration dehydriert und getrocknet.

1.b) In einer weiteren Ausführungsform der Erfindung wird das Cyclodextrinderivat in Dichlormethan, Dichlorethan, Pyridin, Dimethylformamid oder Dimethylsulfoxid, bevorzugt in Dichlormethan oder Dichlorethan, gelöst und als Vernetzungsmittel bevorzugt Epichlorhydrin oder 1,2,9,10-Diepoxy-4,7-dioxadecan zugegeben. Nach dem Rühren wird als Katalysator bevorzugt Trifluoressigsäureanhydrid oder Bortrifluoridetherat zugegeben. Die Cyclodextrinkonzentration in der Lösung beträgt 10 - 50 Gew.-%. Das Molverhältnis des Vernetzungsmittels zum Cyclodextrinderivat beträgt 50 - 20 : 1 , bevorzugt 10 - 15 : 1. Das Reaktionsgemisch wird für weitere 1 - 4 Stunden gerührt und anschließend abkühlen gelassen. Wenn das Produkt löslich ist, wird ein 1 - 5-facher Wasserüberschuß, bezogen auf das Volumen des Reaktionsgemisches, zugegeben und das organische Lösungsmittel abgedampft und die wäßrige Lösung dialysiert und lyophilisiert. Wenn das Produkt ein quellfähiges Polymer ist, wird es mit dem gerade verwendeten organischen Lösungsmittel und anschließend mit Ethanol und/oder Aceton gewaschen und anschließend getrocknet.

2. Herstellung von alkylierten Cyclodextrinpolymeren

Die alkylierten Cyclodextrinpolymere können durch Verbindung der alkylierten Cyclodextrine gemäß den in 1.a) und 1.b) beschriebenen Verfahren hergestellt werden oder durch Alkylierung der Cyclodextrinpolymere. Im letzteren Fall werden 5 - 50 g des Cyclodextrinpolymers gelöst oder in 100 $cm^3$ einer wäßrigen Lösung von 0,1 - 10 Mol/l Alkali- oder Erdalkalihydroxid oder in Pyridin, Dimethylsulfoxid, N,N-Dimethylformamid, enthaltend 0 - 2 Mol Alkalihydroxid pro 1 Mol Alkylierungsmittel, gequollen. Das Alkylierungsmittel (Haloalkane, Alkylsulfate, Alkylepoxide) wird zu dieser Reaktionsmischung gegeben. Das Molverhältnis beträgt 1 - 25 Mole Alkylierungsmittel auf 1 Mol Cyclodextrin im Polymer. Die Alkylierung wird bei Raumtemperatur oder bei einer niedrigeren oder höheren Temperatur ausgeführt. Nach 0,5 - 24 Stunden Reaktionszeit werden die quellfähigen Polymere filtriert, gewaschen und getrocknet, die löslichen Polymere durch Dialyse oder Chromatographie gereinigt und lyophilisiert.

3. Herstellung von acylierten Cyclodextrinpolymeren

Acylierte Cyclodextrinpolymere können durch Verbindung acylierter Cyclodextrine gemäß den in 1.a) und 1.b) beschriebenen Verfahren oder durch Acylierung von Cyclodextrinpolymeren hergestellt werden. Die Acylierung wird in wäßriger Lösung, in organischer Lösung oder in organischer Lösung mit einem minimalen Wassergehalt (Pyridin, Essigsäure, N,N-Dimethylformamid) unter Verwendung organischer oder anorganischer Basen oder Säuren, wasserfreier oder wasser-enthaltender Lewis-Säuren (Aluminiumchlorid, Eisenchlorid, Bortrifluoridetherat, Zinkchlorid) oder ohne Katalyse mit einem geeigneten Säureanhydrid, Acylchlorid, geeigneten Estern oder kurzkettigen Alkoholen oder Cyclodextrinestern, durchgeführt.

Das Produkt kann durch allgemein verwendete Verfahren gereinigt werden :
Die Cyclodextrinmonomere durch Kristallisation,
die Cyclodextrinpolymere durch Waschen oder Dialyse,
beide Produkte durch geeignete chromatographische Verfahren.

Die acylierten Cyclodextrine können während der Polymerisation deacetyliert werden. Der Deacetylationsgrad hängt u.a. vom Substitutionsgrad des Ausgangsmaterials und von den Eigenschaften der Substituenten ab.

4. Herstellung von Stickstoff- oder Schwefel-enthaltenden lipophilen Cyclodextrinpolymeren

Stickstoff- und/oder Schwefel-enthaltende Cyclodextrinpolymere können durch Polymerisation geeigneter Cyclodextrinderivate gemäß dem in 1.a) und 1.b) beschriebenen Verfahren hergestellt werden.

5. Silylierte Cyclodextrinpolymere

Silylierte Cyclodextrinpolymere können durch Verbindung von silylierten Cyclodextrinmonomeren gemäß den in 1.a) und 1.b) beschriebenen Verfahren hergestellt werden oder durch Silylierung von Cyclodextrinpolymeren mit Silylierungsmitteln (Chlortrimethylsilan, 1,1,1,3,3,3-Hexamethyl-disilazan, Tertbutyldimethylsilyl-chlorid) in absoluten organischen Lösungsmitteln (Pyridin, Ether, N,N-Dimethyl-formamid) in Gegenwart oder Abwesenheit organischer oder anorganischer Basen.

Das Produkt kann durch folgende allgemein gebräuchliche Verfahren hergestellt werden :
Die Cyclodextrinmonomere durch Kristallisation,
die Cyclodextrinpolymere durch Waschen oder Dialyse,
beide Produkte durch geeignete chromatographische Verfahren.

Die erfindunsgemäß hergestellten Produkte sind durch ihren Cyclodextringehalt gekennzeichnet, durch ihren Gehalt an Substituenten und durch den geschätzten Substitutionsgrad, bezogen auf den Cyclodextringehalt des Polymeren.

Die Lipophilität der Polymere kann durch ihren Lipophilitätsfaktor gekennzeichnet werden : Das Verhältnis des Sedimentationsvolumens in einem apolaren Lösungsmittel (Benzol) und in einem polaren Lösungsmittel (Wasser). Das Sedimentationsvolumen hängt von der Morphologie der Polymerpartikeln ab, weshalb nur Polymere mit einer ähnlichen Morphologie vergleichbar sind. Der Lipophilitätsfaktor der Cyclodextrinpolymere und der lipophilen Cyclodextrinpolymere beträgt 0,2 - 0,5 bzw. 0,3 - 2,0 .

Die erfindungsgemäß bereitgestellten Produkte behalten ihre Fähigkeit, Einschlußkomplexe zu bilden, ergänzt durch ihre hydrophoben Wechselwirkungen. Diese Produkte können als Sorbentien verwendet werden, um bestimmte Materialen zu adsorbieren und zu konzentrieren. Ihre Sorptionskapazität ist wesentlich höher als diejenigen der nicht-modifizierten Cyclodextrine.

BEISPIEL 1

Wasserlösliches β-Cyclodextrinpolymer aus Dimethyl-β-cyclodextrin

5 g (3,8 mMol) Dimethyl-β-Cyclodextrin wurden in 23 $cm^3$ Dichlorethan bei Raumtemperatur gelöst und hierzu 2,5 $cm^3$ (2,8 g, 1,6 mMol) 1,2,9,10-Diepoxy-4,7-dioxa-decan unter stetigem Rühren zugegeben. 0,25 $cm^3$ (2,2 mMol) Bortrifluoridetherat wurden in 2 $cm^3$ Dichlorethan gelöst und tropfenweise zur Reaktionsmischung zugegeben. Anschließend wurden 0,26 $cm^3$ (2,2 mMol) Bortrifluoridetherat, gelöst in 15 $cm^3$ Dichlorethan, zugegeben. Nach Beendigung der Reaktion wurde die Mischung in 500 $cm^3$ Wasser emulgiert und das Dichlorethan abgedampft. Die niedermolekularen Verunreinigungen wurden durch Dialyse entfernt. Nach der Abdampfung und der Lyophilisation wurden 5 g weißes Pulver erhalten.

Der Cyclodextringehalt, bestimmt durch iodometrische Titration nach saurer Hydrolyse, betrug 58,1 %. Der Gehalt an Methoxygruppen, bestimmt durch iodometrische Titration, betrug 23,1 %, 2,1 Mol pro Glucoseeinheit. Dies stimmt überein mit der geschätzten Zahl an 2 Mol Methoxygruppen pro Mol Glucoseeinheit.

Das Molekulargewicht des Produktes wurde durch seine Eigenviskosität und die gelchromatographische Molekulargewichtsverteilung bestimmt. Die Eigenviskositätszahl betrug 5,7 $cm^3$/g (Wasser 25 ° C).
Die Chromatographiebedingungen waren wie folgt :
Säule : 160 $cm^3$ Ultrogel ACA-34 in einer Pharmacia K 16/100-Säule;
Probe : 300 mg Produkt, gelöst in 5 $cm^3$ destilliertem Wasser
Eluierungsmittel : Destilliertes Wasser
Eluierungsgeschwindigkeit : 30 $cm^3$/min
5 $cm^3$ Proben wurden in einem LKB Ultro Frac-Sammler gesammelt und die optische Aktivität bestimmt. Das auf dem Gelchromatogramm basierende durchschnittliche Molekulargewicht der Probe betrug Mw = 3700, die Polydispersität 1,7.

BEISPIEL 2

Quellfähiges Polymer aus Dimethyl-β-cyclodextrin

2 g (2,6 mMol) Dimethyl-β-cyclodextrin wurden bei Raumtemperatur in 7 $cm^3$ Dichlorethan gelöst und hierzu 1 $cm^3$ (16 mMol) 1,2,9,10-Diepoxy-4,7-dioxa-decan unter stetigem Rühren zugegeben. 0,1 $cm^3$ (1,1 mMol) Bortrifluoridetherat wurden in 1 $cm^3$ Dichlorethan gelöst und tropfenweise zugegeben. Nach 1 Stunde wurden 1 $cm^3$ (16 mMol) 1,2,9,10-Diepoxy-4,7-dioxa-decan und 0,1 $cm^3$ (1,1 mMol) Bortrifluoridetherat, gelöst in 2 $cm^3$ Dichlorethan, hinzugegeben. Das Gel-ähliche Produkt wurde mit Dichlorethan und Methanol gewaschen. Die Ausbeute an Produkt betrug 2,5 g .

Sedimentationsvolumen : 2,0 $cm^3$ /g in Benzol, 2,9 $cm^3$ /g in Wasser.

Die Quellfähigkeit ist gekennzeichnet durch den Lipophilitätsfaktor, der bestimmt ist durch das Vehältnis des Sedimentationsvolumens in Benzol und in Wasser : $V_B/V_W$ = 0,8.

BEISPIEL 3

Lösliches Polymer aus acetyliertem Dimethyl-β-cyclodextrin

1,8 g (1,1mMol) Peracetyl-dimethyl-β-cyclodextrin und 0,2 g (0,1 mMol) Peracetyl-β-cyclodextrin wurden in 3 $cm^3$ Wasser, enthaltend 0,5 g (12 mMol) Natriumhydroxid, bei 60 ° C gelöst. Hierzu wurde 1 $cm^3$ (12 mMol) Epichlorhydrin tropfenweise zugegeben und anschließend 2,5 Stunden lang gerührt. Die Reaktionsmischung wurde anschließend abgekühlt. Die alkalische wäßrige Lösung wurde vom sedimentierten, honig-ähnlichen Produkt dekantiert. Das Produkt wurde in destilliertem Wasser gelöst, Natriumchlorid und Alkali durch Dialyse entfernt und lyophilisiert. Es wurde ein weißes Pulver erhalten, welches in Wasser, Aceton und Benzol löslich war.
Cyclodextringehalt : 64 %
Eigenviskositätszahl : 5,3 $cm^3$ /g
Geschätztes durchschnittliches Molekulargewicht : 3300.

BEISPIEL 4

Methylierung von β-Cyclodextrin-Perlpolymer

1 g β-Cyclodextrin-Perlpolymer (hergestellt gemäß ungarischem Patent 177.419, β-Cyclodextringehalt : 46 %, (0,4 mMol) wurden in 300 $cm^3$ 30 % (2,25 Mol) Natriumhydroxidlösung gequollen und hierzu 56,7 g (0,36 Mol) Dimethylsulfat tropfenweise bei 0 °C zugegeben. Die Reaktionsmischung wurde bei Raumtemperatur 3 Stunden lang gerührt. Das Produkt wurde abgefiltert, von Alkali freigewaschen und bei 105 °C 1 Stunde lang getrocknet.
Methoxygruppengehalt : 11,7 %
Sedimentationsvolumen : 3,1 $cm^3$/g in Benzol, 4,0 $cm^3$/g in Wasser.
Sedimentationsvolumen des Ausgangspolymers : 2,2 bzw. 4,6 $cm^3$/g.
Der Lipophilitätsfaktor nahm von 0,5 auf 0,8 zu.

BEISPIEL 5

Methylierung eines β-Cyclodextrin-Blockpolymeren

3 g eines β-Cyclodextrin-Blockpolymeren (β-Cyclodextringehalt : 48,2 %, 1,28 mMol vernetzt mit 1,2,9,10-Diepoxy-4,7-dioxa-decan) wurden in 120 $cm^3$ einer 30 % (0,9 Mol) Natriumhydroxidlösung bei 5 °C gerührt. Hierzu wurden tropfenweise 42,3 $cm^3$ (0,27 Mol) gekühltes Methylsulfat zugegeben; anschließend wurde die Mischung 1/2 Stunde lang bei 60 °C gerührt. Das Produkt wurde abgefiltert, durch wiederholtes Waschen mit destilliertem Wasser neutralisiert, mit wäßrigen Acetonlösungen zunehmender Konzentration dehydriert und bei 105 °C getrocknet, wobei 3,2 g Produkt erhalten wurde.
Methoxygruppengehalt : 6,2 %
Sedimentationsvolumen : 1,8 $cm^3$/g in Benzol, 3,5 $cm^3$/g in Wasser.
Sedimentationsvolumen des Ausgangspolymeren : 1,1 und 3,3 $cm^3$/g.
Der Lipophilitätsfaktor nahm von 0,3 auf 0,5 zu.

BEISPIEL 6

Methylierung eines β-Cyclodextrin-Blockpolymeren mit einem Gehalt an Polyvinylalkohol

5,5 g eines β-Cyclodextrin-Blockpolymeren (β-Cyclodextringehalt : 48 %, 2,35 mMol Polyvinylalkoholgehalt : 5 %) wurden in 220 $cm^3$ einer 30 % Natriumhydroxidlösung bei -5 °C gerührt und tropfenweise 20,5 $cm^3$ (0,13 Mol) Dimethylsulfat zugegeben. Die Reaktionsmischung wurde 1/2 Stunde lang bei 60 °C gerührt. Das Produkt wurde, wie im Beispiel 5 beschrieben, abgefiltert, gewaschen und dehydriert. 5 g Produkt wurden erhalten.
Methoxygruppengehalt = 15,5 %.
Sedimentationsvolumen : 3,6 $cm^3$/g in Benzol, 4,9 $cm^3$/g in Wasser.
Sedimentationsvolumen des Ausgangspolymeren : 1,9 und 4,5 $cm^3$/g.
Der Lipophilitätsfaktor erhöhte sich von 0,4 auf 0,7.

BEISPIEL 7

Butylierung eines α-Cyclodextrin-Perlpolymeren

10 g eines α-Cyclodextrin-Perlpolymeren (α-Cyclodextringehalt: 57,2 %, 5,9 mMol, Teilchengröße 80 - 250 µm) wurden in 100 $cm^3$ Dimethylsulfoxid bei Raumtemperatur gerührt. 5 g Natriumhydroxidpulver (0,125 Mol) wurden gelöst und während 1 Stunde 5 $cm^3$ (0,044 Mol) 1-Brombutan tropfenweise zugegeben. Die Reaktionsmischung wurde über Nacht gerührt, anschließend abgefiltert, mit verdünnter Salzsäure und Wasser gewaschen, mit Aceton dehydriert und bei 80 °C unter Vakuum getrocknet. 11,2 g Produkt wurden erhalten.
Sedimentationsvolumen : 4,3 $cm^3$/g in Benzol, 5,0 $cm^3$/g in Wasser.
Sedimentationsvolumen des Ausgangspolymeren : 1,3 und 6,0 $cm^3$/g.
Der Lipophilitätsfaktor nahm von 0,3 auf 0,5 zu.

BEISPIEL 8

Hydroxypropylierung eines Gamma-Cyclodextrin-Perlpolymeren

1,2 g (0,03 Mol) Natriumhydroxid wurden in 50 $cm^3$ Wasser gelöst und 10 g Gamma-Cyclodextrin-Perlpolymer (Cyclodextringehalt : 51 %, 3,9 mMol, Teilchengröße 80 - 250 $\mu$m) wurden in dieser Lösung gequollen und hierzu 10 $cm^3$ Propylenoxid (0,148 Mol) zugegeben. Die Reaktionsmischung wurde über Nacht gerührt, anschließend abgefiltert, neutralisiert, dehydriert und getrocknet. 17,1 g Produkt wurden erhalten.
Sedimentationsvolumen : 2,5 $cm^3$/g in Benzol, 4,3 $cm^3$/g in Wasser.
Sedimentationsvolumen des Ausgangspolymeren : 1,9 bzw. 4,8 $cm^3$/g.
Der Lipophilitätsfaktor nahm von 0,4 auf 0,6 zu.

BEISPIEL 9

6-Hydroxyhexyl-$\beta$-Cyclodextrin-Perlpolymer

5,0 g (2,2 Mol) eines $\beta$-Cyclodextrin-Perlpolymeren ($\beta$-Cyclodextringehalt : 51 %, 2,2 mMol, Teilchengröße 100 - 300 $\mu$m) wurden bei 80 °C unter stetigem Rühren in 40 $cm^3$ Wasser, enthaltend 4,0 g ( 0,071 Mol) Kaliumhydroxid, gequollen. 6-Chlor-1-hexanol (4 $cm^3$, 0,035 Mol) wurden zugegeben und für 24 Stunden gerührt. Nach Ende der Reaktion wurde das Polymer abgefiltert, mit Salzsäure gesäuert, mit Wasser zur Neutralität gewaschen, mit Aceton dehydriert und bei 80 °C unter Vakuum getrocknet.
Erhaltenes Trockenmaterial : 8,0 g.
Hydroxyhexylgehalt : 60 % ( bezogen auf die Gewichtszunahme ), d.h. 1,4 Hydroxyhexylgruppen pro Cyclodextrineinheiten;
Sedimentationsvolumen : 4,0 $cm^3$/g in Wasser, 1,5 $cm^3$/g in Benzol.
Der Lipophilitätsfaktor erhöhte sich von 0,4 auf 0,6.

BEISPIEL 10

Acetyliertes $\beta$-Cyclodextrin-Perlpolymer

Verfahren 1.

100 g eines trockenen $\beta$-Cyclodextrin-Perlpolymeren ($\beta$-Cyclodextringehalt : 53,5 %, 47 mMol) wurden in wasserfreiem Pyridin (836 $cm^3$) und in Acetanhydrid (580 $cm^3$, 5,24 Mol) suspendiert. Nach 15minütigem Rühren bei Raumtemperatur wurde die Reaktionsmischung 16 Stunden lang stehen gelassen. Anschließend wurde für weitere 16 Stunden stetig gerührt (einschl. eines Abkühlungszeitraums auf Raumtemperatur) worauf eine fünfstündige Erhitzungsphase bei 60 °C folgte.

Das Polymer wurde abfiltriert, mit Aceton und Wasser gewaschen, mit wasserhaltiger Salzsäure verdünnt und anschließend wieder mit Wasser und Aceton gewaschen.
Trockenmaterialausbeute : 145 g;
Acetylgehalt : 25,5 %, d.h. 16,8 Acetylgruppen pro Cyclodextrineinheiten;
Sedimentationsvolumen : 2,0 $cm^3$/g in Wasser;
2,2 $cm^3$/g in Ethanol;
3,0 $cm^3$/g in Methanol;
3,0 $cm^3$/g in Benzol;
Der Lipophilitätsfaktor erhöhte sich von 0,4 auf 1,5.

Verfahren 2.

10,0 g eines $\beta$-Cyclodextrin-Perlpolymeren ( $\beta$-Cyclodextringehalt : 14,9 mMol, Teilchengröße : 10 - 100 $\mu$m) wurden in Acetanhydrid (40 $cm^3$) bei 25 °C suspendiert und hierzu wasserfreies Eisen-(III)-chlorid (0,32 g, 2 mMol) zur Suspension zugegeben. Die Reaktionsmischung wurde 24 Stunden lang bei Raumtemperatur (25 °C) gerührt. Das Polymer wurde abfiltriert, zweimal zum Neutralisieren mit Wasser (20 $cm^3$) und anschließend zweimal mit Aceton (40 $cm^3$) gewaschen. Das Produkt (11,2)g wurde 6 Stunden lang bei 60 °C über Kaliumhydroxid und anschließend 4 Stunden lang bei 105 °C unter Vakuum getrocknet.
Trockenmaterialausbeute : 9,6 g;

Acetylgehalt : mind. 0,5 %;
Sedimentationsvolumen : 2,5 $cm^3$/g in Wasser;
0,7 $cm^3$/g in Benzol;
Der Lipophilitätsfaktor erhöhte sich von 0,2 auf 0,3.

BEISPIEL 11

Palmitoyliertes β-Cyclodextrin-Perlpolymer

10,0 g eines β-Cyclodextrin-Perlpolymeren (4,5 mMol, β-Cyclodextringehalt: 51%, Teilchengröße : 100 - 300 µm) wurden in abs. Pyridin (50 $cm^3$) 30 Min. lang gequollen; anschließend wurde Palmitoylchlorid (17,5 g, 0,064 Mol) zugegeben und über Nacht gerührt. Das Polymer wurde abfiltriert, mit Wasser (10*50 $cm^3$) und Aceton gewaschen und anschließend bei 80 °C unter Vakuum getrocknet.
Trockenmaterialausbeute : 16,3 g;
Sedimentationsvolumen : 2,2 $cm^3$/g in Wasser;
3,1 $cm^3$/g in Benzol (die Werte für das Ausgangspolymer sind 1,9 $cm^3$/g bzw. 4,8 $cm^3$/g);
Der Lipophilitätsfaktor erhöhte sich von 0,4 auf 1,4.

BEISPIEL 12

Dodecylsulfonyl-β-Cyclodextrin-Perlpolymer

10,0 g eines β-Cyclodextrin-Perlpolymeren ( β-Cyclo-dextringehalt: 46%, Teilchengröße : 90 - 300 µm) wurden in Pyridin (50 $cm^3$) 30 Min. lang gequollen; anschließend wurde Dodecylsulfonylchlorid (8,8 g, 0,03 Mol) zugegeben und über Nacht gerührt. Das Polymer wurde abfiltriert, mit Wasser (10*50 $cm^3$) gewaschen, mit Aceton dehydriert und unter Vakuum getrocknet.
Trockenmaterialausbeute : 17,1 g;
Schwefelgehalt : 1,9 %;
Sedimentationsvolumen : 3,1 $cm^3$/g in Wasser;
2,7 $cm^3$/g in Benzol
Der Lipophilitätsfaktor erhöhte sich von 0,5 auf 0,9.

BEISPIEL 13

N-Octylamino-β-Cyclodextrin-Perlpolymer

10,0 g eines N-Octylamino-desoxy-β-cyclodextrins (DS: 1,1) wurden in 20 % einer wäßrigen Natriumhydroxidlösung (10 $cm^3$, 12,5 mMol) bei 60 °C gelöst. Anschließend wurde Epichlorhydrin (4 $cm^3$) zugegeben, 30 Min. lang bei 60 °C gerührt und anschließend in Paraffinöl (30 $cm^3$) unter stetigem Rühren für weitere 5 Stunden dispergiert.
Das Polymer wurde abfiltriert, zum Neutralisieren mit Wasser gewaschen und mit Aceton dehydriert und anschließend bei 80 °C unter Vakuum getrocknet.
Trockenmaterialausbeute : 10,4 g;
β-Cyclodextringehalt : 41 %
Stickstoffgehalt : 2,1 %;
Sedimentationsvolumen : 3,0 $cm^3$/g in Wasser;
2,3 $cm^3$/g in Benzol
Lipophilitätsfaktor : 0,8

BEISPIEL 14

S-Butylthio-desoxy-β-cyclodextrin-Perlpolymer

S-Butylthio-desoxy-β-cyclodextrin (10,0 g, 5,4 mMol, DS: 0,85) wurden bei 60 °C unter Rühren in 5 % einer wäßrigen Natriumhydroxidlösung (15 $cm^3$, 18,8 mMol) unter einer inerten Atmosphäre gelöst. 1,2,11,12-Diepoxy-4,9-dioxa-dodecan ( 15 $cm^3$, 30 mMol) wurden zugegeben. Nach 10 Stunden wurde die Lösung in Paraffinöl (50 $cm^3$) dispergiert. Das Polymer wurde nach 5 Stunden filtriert, mit Salzsäure gesäuert, zum Neutralisieren mit Wasser gewaschen, mit Aceton dehydriert und bei 80 °C unter Vakuum

getrocknet.
Trockenmaterialausbeute : 10,5 g;
Partikelgröße : 10 - 400 µm;
Cyclodextringehalt : 43 %
Schwefelgehalt : 2,0 %;
Sedimentationsvolumen : 3,1 $cm^3$/g in Wasser;
2,5 $cm^3$/g in Benzol
Lipophilitätsfaktor : 0,8

BEISPIEL 15

Trimethylsilylierung eines β-Cyclodextrin-Perlpolymeren

Verfahren 1.

10,0 g eines β-Cyclodextrin-Perlpolymeren (β-Cyclodextringehalt : 56 %, 4,9 mMol β-Cyclodextrin, Teilchengröße : 10 - 100 µm) wurden in abs. Diethylether (100 $cm^3$) bei 25 °C suspendiert. Trimethylsilylchlorid (4,35 g, 0,040 Mol) wurden auf einmal zugegeben. Zu diesem Reaktionsgemisch wurde bei 25 °C abs. Triethylamin (4,45 g, 0,044 Mol) tropfenweise zugegeben, bis zum Kochen erhitzt, 2 Stunden lang unter Rückfluß und weitere 18 Stunden bei 25 °C gerührt.
Das Polymer wurde abfiltriert, zweimal mit eiskaltem Wasser (20 $cm^3$) und anschließend zweimal mit Aceton (40 $cm^3$) gewaschen.

Das Produkt (14,8 g) wurde über Kaliumhydroxid 6 Stunden lang bei 60 °C und anschließend 4 Stunden lang bei 105 °C unter Vakuum getrocknet.
Trockenmaterialausbeute : 10,13 g;
Siliciumgehalt : mind. 0,5 %
Sedimentationsvolumen : 4,0 $cm^3$/g in Wasser;
1,1 $cm^3$/g in Benzol
Der Lipophilitätsfaktor erhöhte sich von 0,2 auf 0,3.

Verfahren 2.

10,0 g eines β-Cyclodextrin-Perlpolymeren (Ausgangsmaterial vergleiche Beispiel 15, Verfahren 1.) wurden in abs. Pyridin (50 $cm^3$) und 1,1,1,3,3,3-Hexamethyldisilazan (1,91g, 0,012 Mol) bei 25 °C suspendiert. Hierzu wurde Trimethylsilylchlorid (2,15 g, 0,020 Mol) tropfenweise bei 25 °C zugegeben. Die Reaktionsmischung wurden auf 60 °C erhitzt, 2 Stunden lang bei 60 °C und weitere 18 Stunden bei 25 °C gerührt.
Das Polymer wurde abfiltriert, zweimal mit eiskaltem Wasser (20 $cm^3$) und zweimal mit Aceton (40 $cm^3$) gewaschen .
Das Produkt (14,6 g) wurde über Kaliumhydroxid 6 Stunden lang bei 60 °C und weitere 4 Stunden bei 105 °C unter Vakuum getrocknet.
Trockenmaterialausbeute : 10,22 g;
Siliciumgehalt : mind. 0,7 %
Sedimentationsvolumen : 4,0 $cm^3$/g in Wasser;
1,7 $cm^3$/g in Benzol
Der Lipophilitätsfaktor erhöhte sich von 0,2 auf 0,4.

Verfahren 3.

10,0 g eines β-Cyclodextrin-Perlpolymeren (Ausgangsmaterial vergleiche Beispiel 15, Verfahren 1.) wurden in abs. N,N-Dimethylformamid (50 $cm^3$) und 1,1,1,3,3,3-Hexamethyldisilazan (4,04 g, 0,025 Mol) bei 25 °C suspendiert. Die Reaktionsmischung wurde bis zum Kochen erhitzt, 2 Stunden lang unter Rückfluß und weitere 18 Stunden bei 25 °C gerührt.
Das Polymer wurde abfiltriert, zweimal mit eiskaltem Wasser (20 $cm^3$) und zweimal mit Aceton (40 $cm^3$) gewaschen .
Das Produkt (15,2 g) wurde über Kaliumhydroxid 6 Stunden lang bei 60 °C und anschließend 4 Stunden bei 105 °C unter Vakuum getrocknet.
Trockenmaterialausbeute : 10,45 g;

Siliciumgehalt : mind. 1,4 %
Sedimentationsvolumen : 3,7 $cm^3$/g in Wasser;
1,2 $cm^3$/g in Benzol
Der Lipophilitätsfaktor erhöhte sich von 0,2 auf 0,3.

BEISPIEL 16

tertButyl-dimethylsilylierung eines $\beta$-Cyclodextrin-Perlpolymeren

10,0 g eines $\beta$-Cyclodextrin-Perlpolymer ($\beta$-Cyclodextringehalt: 56 %, 4,9 mMol $\beta$-Cyclodextrin, Teilchengröße : 10 - 100 $\mu$m) wurden in abs. Pyridin (40 $cm^3$) bei 25 °C suspendiert. tertButyl-dimethylsilylchlorid (3,77 g, 0,025 Mol) wurden tropfenweise zu abs. N,N-Dimethylformamid (10 $cm^3$) zugegeben, auf 60 °C erhitzt, 2 Stunden bei 60 °C und weitere 18 Stunden bei 25 °C gerührt.
Das Polymer wurde abfiltriert, zweimal mit eiskaltem Wasser (20 $cm^3$) und zweimal mit Aceton (40 $cm^3$) gewaschen .
Das Produkt (12,4 g) wurde über Kaliumhydroxid 6 Stunden lang bei 60 °C und weitere 4 Stunden bei 105 °C unter Vakuum getrocknet.
Trockenmaterialausbeute : 9,71 g;
Siliciumgehalt : mind. 0,5 %
Sedimentationsvolumen : 3,8 $cm^3$/g in Wasser;
1,3 $cm^3$/g in Benzol
Der Lipophilitätsfaktor erhöhte sich von 0,2 auf 0,3.

BEISPIEL 17

Phenolsorption aus wäßriger Lösung

Aus einem in Wasser gequollenen, methylierten $\beta$-Cyclodextrinperlpolymeren (BEISPIEL 4, 2,5g) wurde eine Chromatographiesäule ($V_o$ = 10 $cm^3$) hergestellt. Durch diese Säule wurde eine 0,1 % Phenollösung und nach der Regeneration eine 1 % Phenollösung geschickt. Nach einer anschließenden Regeneration wurde eine 3 % Phenollösung durch die Säule geschickt. 10 $cm^3$-Fraktionen wurden gesammelt. Der Phenolgehalt wurde durch Spektrophotometrie bei 267 nm bestimmt. Dieses Verfahren wurde mit dem Ausgangs-$\beta$-Cyclodextrin-Perlpolymeren ($V_o$ = 11,7 $cm^3$) wiederholt. Das sorbierte Phenol/ $\beta$-Cyclodextrin-Molverhältnis, das sorbierte Phenol-/1g $\beta$-Cyclodextrin-Perlpolymer-Verhältnis und das Volumen der Phenol-freien Fraktionen (vermindert um $V_o$) sind in der nachfolgenden Tabelle aufgeführt.

| Phenol (Ph0H) | Ausgangs-$\beta$CD-Perlpolymer | | | Methyliertes-$\beta$CD Perlpolymer | | |
|---|---|---|---|---|---|---|
| Konzentr. (%) | PhOH/$\beta$CD Molverh. | PhOH/Perlpolymer (g/g) | PhOH frei Vol. ($cm^3$) | PhOH/$\beta$CD Molverh. | PhOH/Perlpolymer (g/g) | PhOH frei Vol. ($cm^3$) |
| 0,1 | 0,53 : 1 | 0,02 | 28 | 0,78 : 1 | 0,03 | 55-60 |
| 1 | 4,52 : 1 | 0,17 | 13 | 8 : 1 | 0,30 | 40 |
| 3 | 7,57 : 1 | 0,28 | 3 | 23 : 1 | 0,87 | 35 |

BEISPIEL 18

p-Cresol-Sorption aus wäßrigen Lösungen

Eine p-Cresollösung (0,1%) wurde durch eine nach BEISPIEL 17 hergestellte Säule geschickt. Der p-Cresol-Gehalt wurde durch Spektrophotometrie bei 266 nm gemessen.
Im Fall des $\beta$-Cyclodextrin-Perlpolymeren :
$V_o$ = 11,7 $cm^3$;
Sorbiertes p-Cresol/-$\beta$-Cyclodextrin-Molverhältnis : 0,61 : 1;
p-Cresol-freies-Volumen (vermindert um $V_o$): 43 $cm^3$.
Im Fall des methylierten-$\beta$-Cyclodextrin-Perlpolymeren :

$V_o$ = 10 $cm^3$ ;
Sorbiertes p-Cresol/β-Cyclodextrin-Molverhältnis : 1,1 : 1;
p-Cresol-freies-Volumen (vermindert um $V_o$) : 105 $cm^3$ .

BEISPIEL 19

Benzol-Sorption aus wäßrigen Lösungen

Eine Benzollösung (0,04 %) wurde durch eine nach BEISPIEL 17 hergestellte Säule geschickt. Der Benzol-Gehalt wurde durch Spektrophotometrie bei 247 nm gemessen.
Im Fall des β-Cyclodextrin-Perlpolymeren :
$V_o$ = 11,7 $cm^3$;
Sorbiertes Benzol/β-Cyclodextrin-Molverhältnis : 0,54 : 1;
Benzol-freies-Volumen (vermindert um $V_o$): 38 $cm^3$.
Im Fall des methylierten-β-Cyclodextrin-Perlpolymeren :
$V_o$ = 10 $cm^3$ ;
Sorbiertes Benzol/β-Cyclodextrin-Molverhältnis : 0,63 : 1;
Benzol-freies-Volumen (vermindert um $V_o$) : 90 $cm^3$ .

Beispiel 20

2 g Polymere (A : β-Cyclodextrinpolymer (BCDP); B:Diethylenaminoethyl-BCDP (DEAE-BCDP); C: Carboxymethyl-BCDP (CM-BCDP)) wurden in 8 ml einer wäßrigen Ethanollösung (50 %v/v), mit einem Gehalt an 1,25 % Salicylsäure gequollen, zwei Stunden lang geschüttelt, bei Raumtemperatur über Nacht und anschließend bei 105 °C weitere zwei Stunden lang getrocknet. Die Erzeugnisse sind gekennzeichnet durch ihren Gehalt an Salicylsäure und ihrer Auflösungseigenschaften.

Der Salicylsäuregehalt wurde bestimmt durch wiederholtes Kochen der 0,25 g Probe in 20 ml Phosphatpufferlösung (pH = 7,2), Filtrierung und anschließender photometrischer Messung der Konzentration im abgekühlten Filtrat bei λ = 296 nm. Dieses Verfahren wurde vier Mal wiederholt und die Summe der Ergebnisse errechnet.

Die Auflösungsgeschwindigkeit wurde durch Rühren von 0,2 g der Probe mit einem Magnetrührer in 20 ml destilliertem Wasser oder in einer Phosphatpufferlösung von pH = 7,2 bei 37 °C bestimmt. Proben aus dem Lösungsüberstand wurden zu bestimmten Zeiten (im Bereich von 1 - 60 Min) entnommen und die Konzentration spektrophotometrisch bestimmt. Die Erzeugnisse sind gekennzeichnet durch die gelöste Menge in 60 Min, ausgedrückt in Prozent der Gesamtaufnahme an aktivem Bestandteil.

| Beschreibung des β-CD-Polymers | | | Eigenschaften der Produkte | | |
|---|---|---|---|---|---|
| ionische Gruppe | Gehalt an ionischer Gruppe (meq/g) | Quellung (ml/g) | Salicylsäure Gehalt (%) | gelöste Menge in Wasser in 60 Min. (%) | gelöste Mengein Puffer in 60 Min. (%) |
| - | - | 5,0 | 4,3 | 54 | 81 |
| DEAE | 0,43 | 4,0 | 4,1 | 18 | 61 |
| CM | 0,62 | 3,6 | 4,4 | 58 | 99 |

Beispiel 21

1,2 g Ethacridinlactat werden in 90 ml 50% (v/v) Ethanol gelöst. 6 ml dieser Lösung werden zu 1,5 g β-Cyclodextrinpolymer zum Quellen gegeben. Die Suspension wird 1 Stunde lang geschüttelt, an der Luft zum Trocknen stehengelassen und anschließend bei 105 °C zwei Stunden lang getrocknet.

Der Gehalt an Ethacridinlactat wird gem. Beispiel 1 bestimmt mit der Ausnahme, daß die Probe in einer Lösung aus etwa 0,1 g Benzoesäure in 50 % (v/v) Ethanol gekocht und anschließend spektrophotometrisch bei λ = 370 nm gemessen wird. Die Auflösungsgeschwindigkeit wird, wie im Beispiel 1 beschrieben, bestimmt.

| Beschreibung des β-CD-Polymers | | | Eigenschaften der Produkte | |
|---|---|---|---|---|
| ionische Gruppe | Gehalt an ionischer Gruppe (meq/g) | Quellung (ml/g) | Ethacridinlactat-Gehalt (%) | gelöste Menge in Wasser in 60 Min. (%) |
| - | - | 5,0 | 5,0 | 86 |
| CM | 0,04 | 5,0 | 5,2 | 70 |
| CM | 0,07 | 3,3 | 5,3 | 72 |
| CM | 0,09 | 3,7 | 4,9 | 61 |
| CM | 0,11 | 4,7 | 5,3 | 50 |
| CM | 0,17 | 4,3 | 5,2 | 27 |
| CM | 0,22 | 4,4 | 5,4 | 20 |
| CM | 0,35 | 4,8 | 5,4 | 7,7 |
| CM | 0,62 | 3,6 | 5,2 | 3,5 |
| CM | 0,73 | 3,1 | 4,7 | 1,5 |

Beispiel 22

0,24 g Methylenblau werden in 90 ml 50 % (v/v) Ethanol gelöst. 20 ml dieser Lösung werden zu 5 g β-Cyclodextrinpolymer zum Quellen gegeben. Die Suspension wird 2 Stunden lang geschüttelt, an der Luft zum Trocken stehengelassen und anschließend bei 105 °C getrocknet.
Die Produkte werden wie im Beispiel 2 beschrieben bestimmt, wobei die spektrophotometrischen Messungen bei λ = 661 nm durchgeführt werden.

| Beschreibung des β-CD-Polymers | | | Eigenschaften der Produkte | |
|---|---|---|---|---|
| ionische Gruppe | Gehalt an ionischer Gruppe (meq/g) | Quellung (ml/g) | Methylenblau Gehalt (%) | gelöste Menge in Wasser in 60 Min. (%) |
| - | - | 5,0 | 1,2 | 34 |
| CM | 0,06 | 4,8 | 1,3 | 32 |
| CM | 0,62 | 3,6 | 1,2 | 9,2 |

## Patentansprüche

**1.** Einschlußkomplexe aus polymerisierten, unlöslichen und in Wasser quellbaren Cyclodextrinen und/oder deren Derivaten mit Desinfizientia, Antiseptika, Antimykotika, Antiphlogistika, Antibiotika oder Anästhetika oder deren Mischungen als Wirkstoffe.

**2.** Einschlußkomplexe nach Anspruch 1,
dadurch gekennzeichnet,
daß das Cyclodextrinpolymer ein α-Cyclodextrin, ein β-Cyclodextrin oder ein γ-Cyclodextrin oder ein Derivat dieser Cyclodextrine ist.

**3.** Einschlußkomplexe nach Anspruch 1 oder 2,
dadurch gekennzeichnet,

daß das Cyclodextrinpolymer ein β-Cyclodextrin oder ein β-Cyclodextrin-Derivat ist.

**4.** Einschlußkomplexe nach einem oder mehreren der vorhergehenden Ansprüche, dadurch gekennzeichnet, daß ionische oder nicht-ionische Cyclodextrinderivate verwendet werden, wobei die ionische Gruppe insbesondere eine Carboxyalkyl-, Sulphalkyl-, Alkylamino- und/oder Dialkylaminogruppe ist, wobei die Alkylgruppe bevorzugt ein $C_1$ - $C_5$-Alkylrest ist und wobei die nicht-ionische Gruppe insbesondere eine Alkyl-, Arylalkyl-, Acyl-, Alkylsulphonyl-, Alkylthio-, Desoxyaminoalkyl- und/oder Silylgruppe ist, wobei der Alkylrest bevorzugt ein $C_1$ -$C_5$-Alkylrest ist.

**5.** Einschlußkomplexe nach einem oder mehreren der vorhergehenden Ansprüche, dadurch gekennzeichnet, daß der Alkylrest ein verzweigter und/oder unverzweigter $C_1$ - $C_5$-Alkylrest, bevorzugt ein $C_1$ - $C_3$-Alkylrest, ist.

**6.** Einschlußkomplexe nach einem oder mehreren der vorhergehenden Ansprüche, dadurch gekennzeichnet, daß die polymeren Cyclodextrine mehr als 5 Cyclodextrineinheiten enthalten.

**7.** Einschlußkomplexe nach einem oder mehreren der vorhergehenden Ansprüche, dadurch gekennzeichnet, daß die polymeren Cyclodextrine, gemessen durch iodometrische Titration der reduzierenden Endgruppen, 30 - 70 %, bevorzugt 50 - 60 % Cyclodextrin enthalten.

**8.** Einschlußkomplexe nach einem oder mehreren der vorhergehenden Ansprüche, dadurch gekennzeichnet, daß der Substitutionsgrad (Zahl der Substituenten/Glucoseeinheit) im Bereich von 0 - 3, bevorzugt im Bereich von 0 - 2 liegt, insbesondere daß der Substitutionsgrad im Bereich von 0,01 - 2, bevorzugt im Bereich 0,01 - 1,5 liegt, wobei insbesondere der Substitutionsgrad bei kleineren Substituenten wie $C_1$ - $C_3$-Alkyl- und Arylsubstituenten im Bereich von 0,01 - 2, bei größeren Substituenten wie Acetylpolymersubstituenten im Bereich von 0,01 - 1,5 liegt.

**9.** Einschlußkomplexe nach einem oder mehreren der vorhergehenden Ansprüche, dadurch gekennzeichnet, daß lipophile Cyclodextrinpolymerderivate der folgenden allgemeinen Formel verwendet werden:

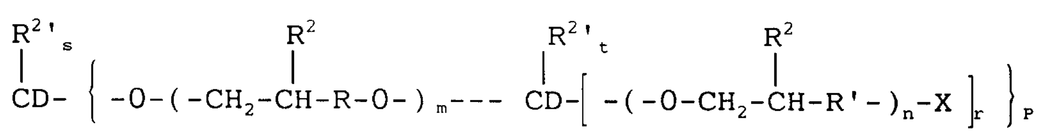

wobei
CD aus einem α-, β- und/oder γ-Cyclodextrinmolekül durch Abspaltung von p+s- oder 1+t+r-Hydroxylgruppen stammt,
R und R'unabhängig voneinander aus der folgenden Gruppe ausgewählt werden :
$-CH_2-$ $-CH0H-CH_2-$ $-CH_2-0-(CH_2)_2-0-CH_2-CH0H-CH_2-$ , $-CH_2-0-CH_2-CHOH-CH_2-$ or $-CH_2-0-(CH_2)_4-0-CH_2-CHOH-CH_2-$
X $OR^1$ oder $R^2$ bedeutet, wobei
$R^1$ ein Wasserstoffatom oder - falls r nicht 0 ist - eine Gruppe darstellt, welche durch die Abspaltung eines Wasserstoffatoms aus einer Hydroxylgruppe aus einem α-, β- oder γ-Cyclodextrinmolekül erhalten wird.
$R^2$ gleiche oder verschiedene Bedeutungen aufweist, nämlich $0R^3$ oder eine Alkylthio-, Alkylsulfonyl-, Alkylsulfinyl- oder Aminoalkylgruppe mit 1-20 Kohlenstoffatomen, bevorzugt mit 1 bis 6 C-Atomen, oder eine Gruppe darstellt, welche durch die Abspaltung eines Wasserstoffatoms aus einer Hydroxylgruppe aus einem α-, β- oder γ-Cyclodextrinmolekül erhalten werden,
$R^{2'}$ der Bedeutung von $R^2$ entspricht, jedoch keine der Gruppen darstellt, die aus den Cyclodextrinmolekülen erhalten wurden, welche durch die Abspaltung eines Wasserstoffatoms aus einer Hydroxylgruppe aus einem α-, β- oder γ-Cyclodextrinmolekül erhalten wird,
$R^3$ eine Alkyl-Gruppe mit 1 - 20 C-Atomen, bevorzugt mit 1 bis 6 C-Atomen, oder eine Aralkyl-oder Alkylarylgruppe darstellt, wobei die Arylgruppe aus einer aromatischen oder heteroaromatischen Verbindung abstammt, oder $R_3$ eine $C_1$ - $C_7$-Acylgruppe oder eine unsubstituierte oder $C_1$ - $C_5$-mono-, di- oder trisubstituierte Silylgruppe darstellt.
m und n unabhängig voneinander ein ganzzahliges von 1 - 10 sind,
r ein ganzzahliges von 0 - 23 ist,
p ein ganzzahliges von 0 - 24 ist,
s und t unabhängig voneinander ein ganzzahliges von 0 - 7 sind,
m, n, r und t, einschließlich in den Seitenketten, innerhalb einer Einheit je unterschiedlich sein können,
und, falls CD eine Gruppe ist, die aus einem α-Cyclodextrin stammt, $p+s<18$ und $r+t<17$ sind,
und, falls CD eine Gruppe ist, die aus einem β-Cyclodextrin stammt, $p+s<21$ und $r+t<20$ sind,
und, falls CD eine Gruppe ist, die aus einem gamma-Cyclodextrin stammt, $p+s<24$ und $r+t<23$ sind.

**10.** Einschlußkomplexe nach Anspruch 9,
dadurch gekennzeichnet,
daß die Alkylgruppen sowohl n-Alkylgruppen als auch ihre Isomere, bevorzugt $C_1$ - $C_{20}$-Alkylgruppen, insbesondere bevorzugt $C_1$ - $C_{10}$-Alkylgruppen umfassen, daß die Arylgruppen insbesondere Phenyl-, Naphthyl-, Biphenyl-, Anthracyl-Gruppen umfassen,
daß die Alkylarylgruppen insbesondere Tolyl-, Xylyl-, Ethylphenyl-, Propylphenyl-, tertiäre Butylphenyl-Gruppen umfassen,
daß die Aralkylgruppen insbesondere Benzyl-, Phenethyl-, Benzhydryl- und Trityl-Gruppen umfassen,
daß die Heteroarylgruppen insbesondere Piridyl-, Picolyl-, Bipyridyl- und Collidyl-Gruppen umfassen,
daß die Acylgruppen insbesondere Formyl-, Acetyl-, Propionyl-, Butyryl-, Isobutyryl-, Valeryl-, Isovaleryl-, Stearyl-, Oleyl- und Palmitoyl-Gruppen umfassen,
daß die S-enthaltenden Gruppen insbesondere Methylsulphonyl-, Ethylsulphonyl-, Hexylsulphonyl-, Dodecylsulphonyl-, Methylthio-, Butylthio-, Hexylthio-Gruppen umfassen,
daß die N-enthaltenden Gruppen insbesondere Amino-, Methylamino-, Ethylamino-, Propylamino-, Hexylamino-, Dodecylamino- und Imidazoyl-Gruppen umfassen und
daß Silylgruppen insbesondere Trimethylsilyl- und tertiäre Butyldimethylsilyl-Gruppen umfassen.

**11.** Einschlußkomplexe nach Anspruch 9,
dadurch gekennzeichnet,
daß das Cyclodextrin ein β-Cyclodextrinmolekül ist.

**12.** Einschlußkomplexe nach Anspruch 9,
dadurch gekennzeichnet,
daß m und n unabhängig voneinander ein Ganzzahliges von 3 bis 7 sind und/oder
r ein Ganzzahliges von 5 bis 17 oder von 8 bis 14 ist, und/oder
b ein Ganzzahliges von 5 bis 17 oder von 8 bis 14 ist, und/oder
s und t unabhängig voneinander ein Ganzzahliges von 2 bis 5 sind und/oder
m, n, r und t, einschließlich in den Seitenketten, innerhalb einer Einheit je unterschiedlich sein können,
und, falls CD eine Gruppe ist, die aus einem α-Cyclodextrin stammt, $p+s<13$ und $r+t<14$ sind oder $p+s<10$ und $r+t<10$ sind,
und, falls CD eine Gruppe ist, die aus einem β-Cyclodextrin stammt, $p+s<17$ und $r+t<16$ sind und/oder $p+s<10$ und $r+t<9$
und, falls CD eine Gruppe ist, die aus einem gamma-Cyclodextrin stammt, $p+s<20$ und $r+t<19$ sind und/oder $p+s<14$ und $r+t<13$ sind.

**13.** Einschlußkomplexe nach einem oder mehreren der vorhergehenden Ansprüche,
dadurch gekennzeichnet,
daß die Antiseptika Salicylsäure und ihre Abkömmlinge, Chloramphenicol, Chamazulen, Iod, Phenol, Thymol, Benzoesäure, Benzalkoniumchlorid, Resorcin, Cetylpyridiniumsalze, Anilinfarbstoffe und/oder Akridinderivate sind, wobei die Anilinfarbstofffe Fuchsin, Gentianaviolett,
Brilliantgrün und/oder Methylenblau sind,
daß die Antiphlogistika Azulen, Hydrocortison, Prednisolon, Depersolon und/oder Triamcinolon sind,
daß die Anästhetika Lodocain, Tetracain, Chlorprocain, Cyclomethycain, Benzocain, Xylocain, Etidocain,

Oxycain und/oder Procain sind,
daß die Antibiotika Methycillin, Oxacillin, Semicillin, Pyrazocillin, Streptomycin, Dihydrostreptomycin, Gentamycin, Chloramphenicol, Oxytetracyclin und/oder Biomycin sind und
daß die Antimykotika Nystatin, Amphotericin B, Tolnaftat, Miconazol und/oder Ketoconazol sind.

**14.** Einschlußkomplexe nach einem oder mehreren der vorhergehenden Ansprüche,
dadurch gekennzeichnet,
daß die Wirkstoffe eine Mischung aus antiseptisch wirkenden und entzündungshemmenden Wirkstoffen sind oder
daß die Wirkstoffe eine Mischung aus antiseptisch und lokalanästhetisch wirkenden Wirkstoffen sind.

**15.** Einschlußkomplexe nach einem oder mehreren der vorhergehenden Ansprüche,
dadurch gekennzeichnet,
daß das Gewichtsverhältnis Cyclodextrin zu Wirkstoff im Bereich von 0,001 - 20 % Wirkstoff und 80 - 99,9 % Cyclodextrin liegt,
daß das Gewichtsverhältnis Cyclodextrin zu Wirkstoff vorzugsweise im Bereich von 1 - 5 % Wirkstoff und 95 - 99 % Cyclodextrin liegt und
daß das Gewichtsverhältnis Cyclodextrin zu Wirkstoff insbesondere 2 % Wirkstoff und 98 % Cyclodextrin beträgt.

**16.** Einschlußkomplexe nach einem oder mehreren der vorhergehenden Ansprüche,
dadurch gekennzeichnet,
daß die Wirkstoffe ausgewählt werden aus einer oder mehreren der Verbindungen der Gruppe, bestehend aus den Farbstoffen basischem Fuchsin, saurem Fuchsin, Brilliantgrün, Gentianaviolett, Methylenblau, Eosin, Ethacridinlactat und Kristallviolett.

**17.** Einschlußkomplexe nach einem oder mehreren der vorhergehenden Ansprüche,
dadurch gekennzeichnet,
daß sie ein $\beta$-Cyclodextrinpolymer oder $\beta$-Cyclodextrinpolymerderivat und Nystatin als Wirkstoff enthalten.

**18.** Einschlußkomplexe nach einem oder mehreren der vorhergehenden Ansprüche,
dadurch gekennzeichnet,
daß sie ein $\beta$-Cyclodextrinpolymer oder $\beta$-Cyclodextrinpolymerderivat und Amphotericin B als Wirkstoff enthalten.

**19.** Verfahren zur Herstellung der Einschlußkomplexe nach einem oder mehreren der vorhergehenden Ansprüche,
dadurch gekennzeichnet,
daß
a) $\alpha$) das Cyclodextrin bzw. sein Derivat in einer geeigneten Lösung des Wirkstoffs bzw. der Wirkstoffe gequollen wird oder
$\beta$) das Cyclodextrin oder sein Derivat mit dem Wirkstoff bzw. den Wirkstoffen vermischt und anschließend in einer geeigneten Lösung gequollen wird,
b) diese Suspension nach Beendigung der Auflösung des Wirkstoffs bzw. der Wirkstoffe und der Quellung falls notwendig filtriert und anschließend getrocknet wird.

**20.** Verfahren zur Herstellung der Einschlußkomplexe nach einem oder mehreren der vorhergehenden Ansprüche,
dadurch gekennzeichnet,
daß zur Auflösung der Wirkstoffe und zum Quellen eine wäßrige oder eine wäßrig-alkoholische Lösung oder eine Pufferlösung verwendet wird.

**21.** Verfahren zur Herstellung der Einschlußkomplexe nach einem oder mehreren der vorhergehenden Ansprüche, dadurch gekennzeichnet,
daß die Suspension nach Beendigung der Auflösung der Wirkstoffe und der Quellung - je nach den Eigenschaften der verwendeten Wirkstoffe - bei Zimmertemperatur oder bei erhöhten Temperaturen getrocknet wird.

**22.** Verfahren zur Herstellung der Einschlußkomplexe nach einem oder mehreren der vorhergehenden Ansprüche,
dadurch gekennzeichnet,
daß die Auflösung der Wirkstoffe und die Quellung bei Temperaturen von 0 - 60 °C, bevorzugt bei Zimmertemperatur, für 0,5 - 24 Stunden, bevorzugt für 1 - 6 Stunden, durchgeführt wird.

**23.** Verwendung der Einschlußkomplexe nach einem oder mehreren der vorhergehenden Ansprüche in einer pharmazeutischen Zubereitung mit kontrollierter Freisetzung der Wirkstoffe.

**24.** Verwendung der Einschlußkomplexe nach einem oder mehreren der vorhergehenden Ansprüche in einer pharmazeutischen Zubereitung zur Behandlung infizierter Wunden, Geschwüre, traumatischer Verletzungen und Verbrennungen unter Ausnutzung sowohl der Drainagewirkung der Cyclodextrinmoleküle als auch der kontrollierten Freisetzung der Wirkstoffe aus den Cyclodextrinmolekülen.

**25.** Verwendung der Einschlußkomplexe nach einem oder mehreren der vorhergehenden Ansprüche in einem Wundpuder.

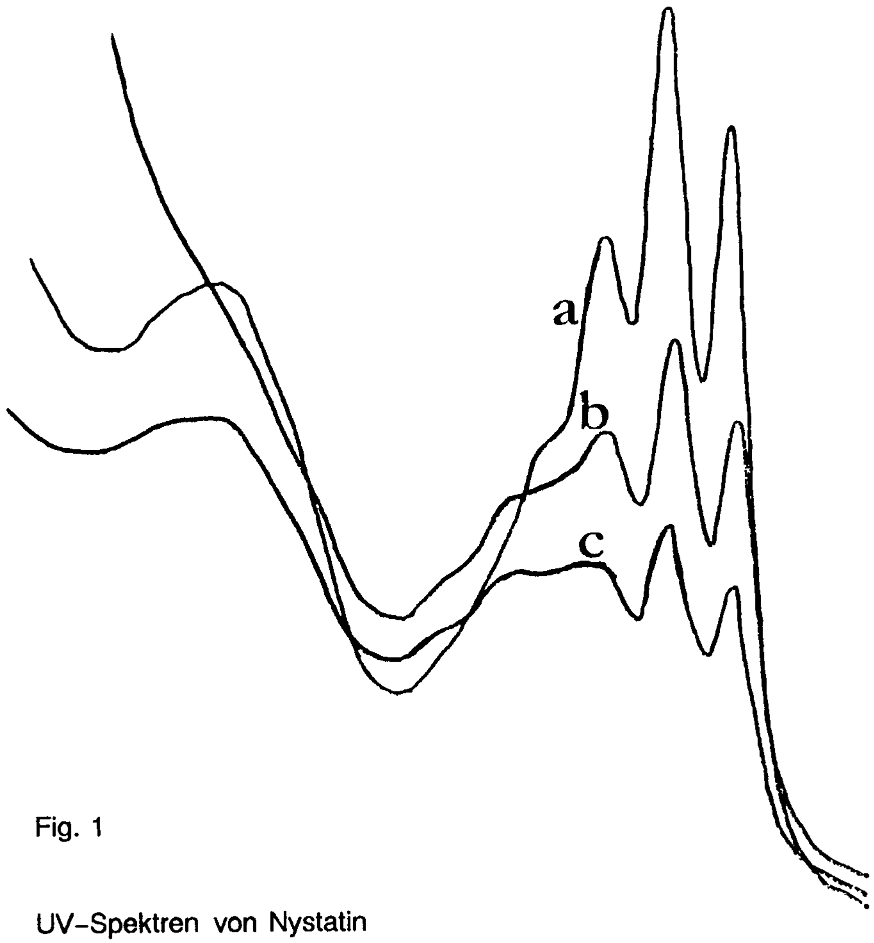


Fig. 1

UV-Spektren von Nystatin

a: in 50% Ethanol

b: in wäßriger Lösung

c: in β-Cyclodextrin enthaltender Lösung

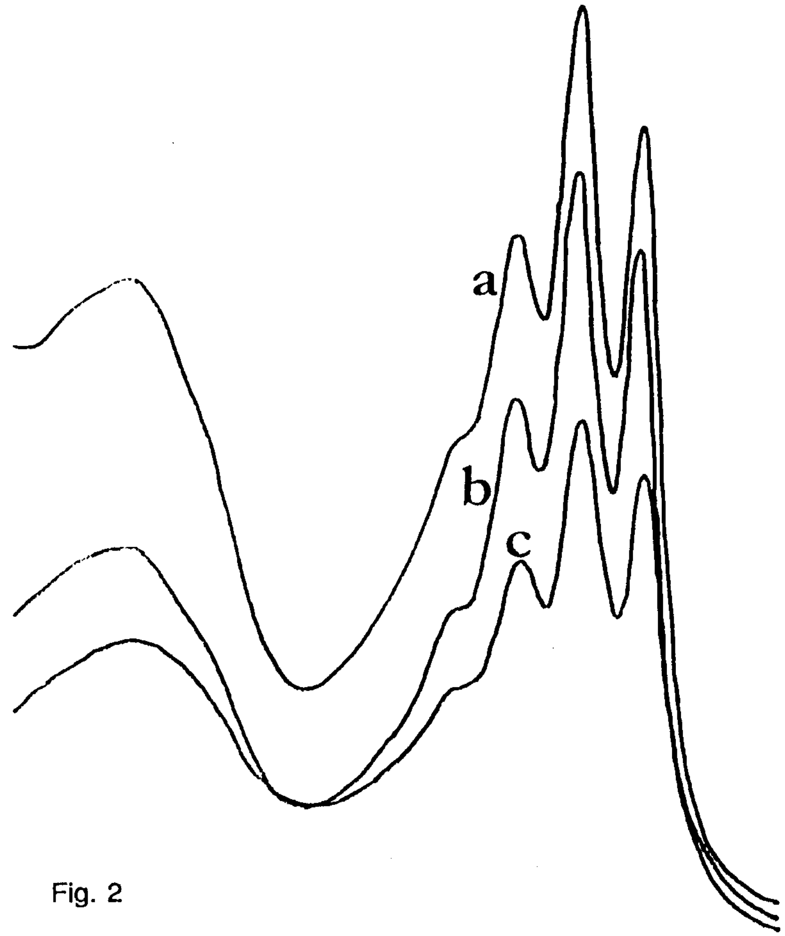


Fig. 2

UV-Spektren von Nystatin, aufgelöst in β-Cyclodextrinpolymer

a: unsubstituiertes Polymer

b: methyliertes Polymer

c: γ-Cyclodextrin komplexiert als Referenz

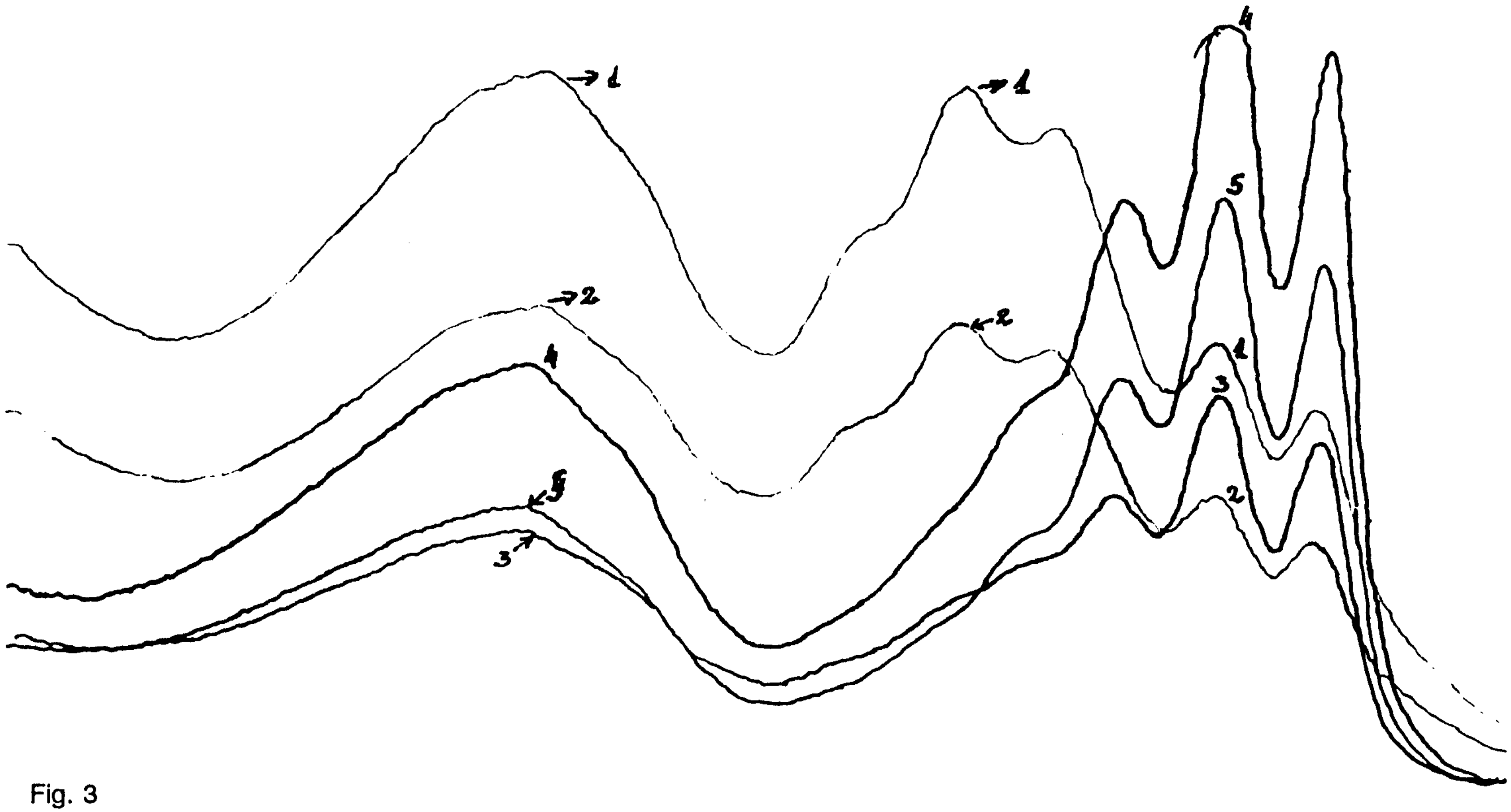

Fig. 3

Absorptionsspektren von Nystatin in destilliertem Wasser (1) in Gegenwart von 1,6% βCD (2), 2,5% (3), 5,0% (4) und 10% γCD (5).

Verdünnungen: 20 (1), 40 (2), 200 (3), 200 (4) und 500 (5) Mal mit destilliertem Wasser

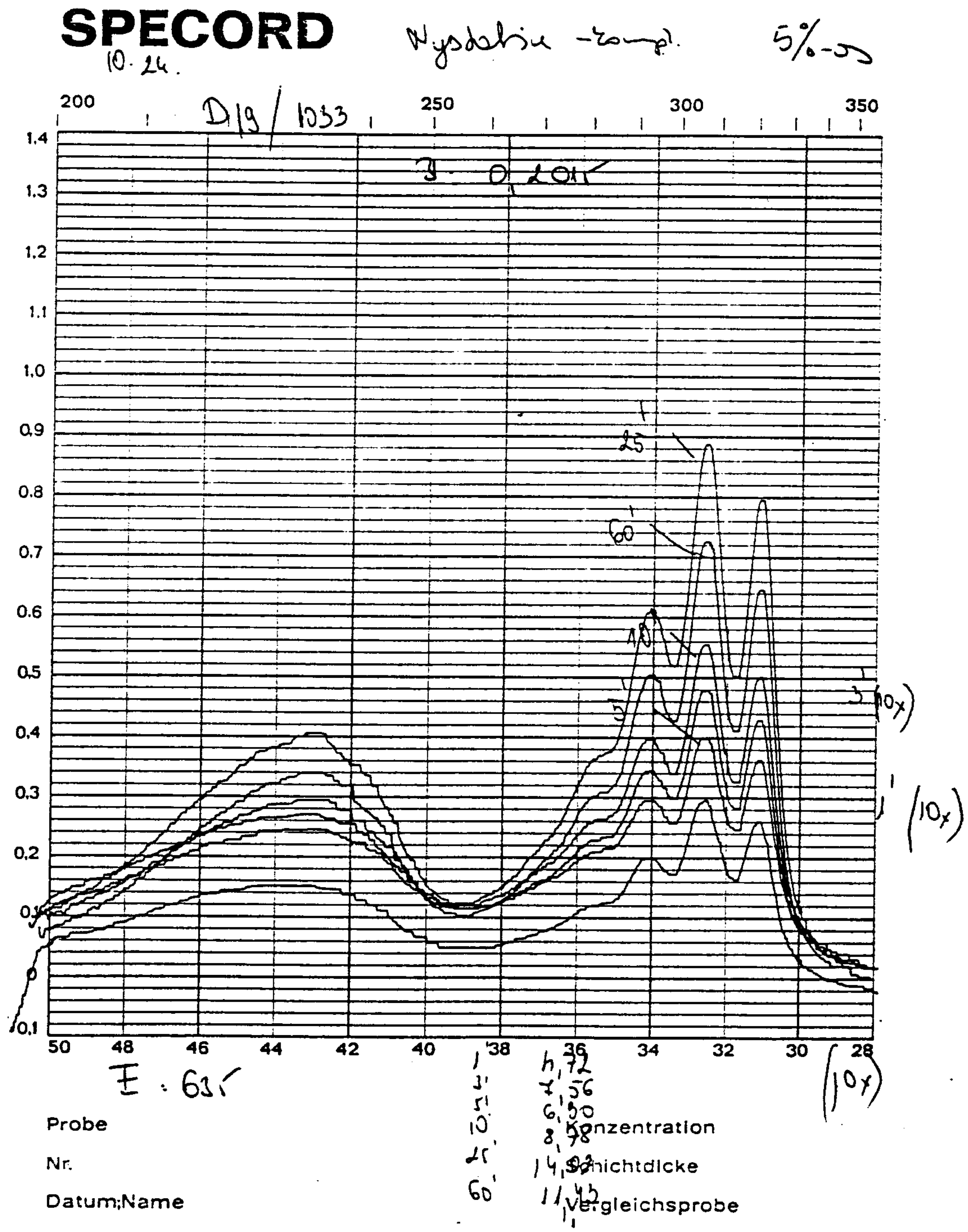


Fig. 4

Absorptionsspektren von Nystatin, gelöst aus $\beta$CD-Polymeren, vernetzt mit Butylenglycol- bis (Epoxypropyl)ether nach 1, 3, 5, 10, 25, und 60 Min.

Europäisches Patentamt

# EUROPÄISCHER RECHERCHENBERICHT

Nummer der Anmeldung

EP 93 11 0009
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X,Y | EP-A-0 119 453 (CHINOIN) 26. September 1984 * Seite 5, Zeile 5 - Seite 7, Zeile 34 * * Seite 3, Zeile 7 - Zeile 22 * --- | 1-25 | A61K47/48 C08B37/00 |
| X,Y | FR-A-2 579 460 (CHINOIN) 3. Oktober 1986 * Seite 2, Zeile 34 - Zeile 36; Ansprüche 1,6,12; Beispiele 6-9; Tabelle II * --- | 1-25 | |
| X | DE-A-3 819 498 (CONSORTIUM FÜR ELEKTROCH. INDISTRIE GMBH) 29. Dezember 1988 * Ansprüche; Beispiele * --- | 1-25 | |
| X,Y | WO-A-9 002 141 (AUSTRALIAN COMMERCIAL RES. & DEV. LTD.) 8. März 1990 * Seite 1, Absatz 1 * * Seite 6, Absatz 2 * * Seite 13 * * Seite 20 * * Seite 23, Absatz 2; Anspruch 1 * --- | 1-25 | **RECHERCHIERTE SACHGEBIETE (Int. Cl.5 )** A61K C08B |
| X | GB-A-2 207 865 (BIOGAL GYOGYZERGYAR) 15. Februar 1989 * Seite 7, Absatz 1; Ansprüche 1,8 * --- | 1-25 | |
| D,X | US-A-4 774 329 (R. B. FRIEDMAN) 27. September 1988 * Spalte 4, Zeile 32 - Zeile 39; Ansprüche; Beispiele 1,3 * --- | 1-25 | |
| Y | WO-A-9 114 710 (CONSORTIUM FÜR ELEKTROCHEM. INDUSTRIE GMBH) 3. Oktober 1991 * Seite 6, Absatz 2 * * Seite 10, Absatz 3; Ansprüche 1,6 * --- -/-- | 1-25 | |

Der vorliegende Recherchenbericht wurde für alle Patentansprüche erstellt

| Recherchenort | Abschlußdatum der Recherche | Prüfer |
|---|---|---|
| DEN HAAG | 11 AUGUST 1993 | BERTE M.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 11 0009


## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, vol. 102, no. 12<br>Columbus, Ohio, US;<br>abstract no. 100693,<br>* Zusammenfassung *<br>& J. INCLUSION PHENOM.<br>Bd. 1, Nr. 4, 1984,<br>Seiten 395 - 402<br>T. CSERHATI ET AL 'INCLUSION COMPLEXING BY WATER-SOLUBLE BETA-CYCLODEXTRIN POLYMERS.'<br>--- | 1-25 | |
| X | CHEMICAL ABSTRACTS, vol. 98, no. 24<br>Columbus, Ohio, US;<br>abstract no. 204299,<br>* Zusammenfassung *<br>& J. CHROMATOGR.<br>Bd. 259, Nr. 1, 1983,<br>Seiten 107 - 110<br>T. CSERHATI ET AL. 'EFFECT OF WATER-SOLUBLE BETA-CYCLODEXTRIN POLYMER ON THE LIPOPHILCITY OF POLYMYXIN'<br>--- | 1-25 | RECHERCHIERTE SACHGEBIETE (Int. Cl.5) |
| X | CHEMICAL ABSTRACTS, vol. 112, no. 24<br>Columbus, Ohio, US;<br>abstract no. 219101y,<br>* Zusammenfassung *<br>& PROC. INT. SYMP. CYCLODEXTRINS<br>Bd. 4, 1988, (DORDRECHT)<br>Seiten 227 - 235<br>E. FENYVESI ET AL. 'COMPLEXES OF INSOLUBLE CYCLODEXTRIN POLYMERS.'<br>----- | 1-25 | |

Der vorliegende Recherchenbericht wurde für alle Patentansprüche erstellt

| Recherchenort | Abschlußdatum der Recherche | Prüfer |
|---|---|---|
| DEN HAAG | 11 AUGUST 1993 | BERTE M.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)